# EUROPEAN PATENT SPECIFICATION

(11) **EP 3 787 594 B1**
(45) Date of publication and mention of the grant of the patent: **31.08.2022**
(21) Application number: 19727169.5
(22) Date of filing: 30.04.2019
(51) Int. Cl.: A61K 8/891, A61Q 19/00, A61K 8/895, C08G 77/42, C08G 77/44, C08L 83/06, C08G 77/14, C08G 77/38

(54) **CROSS-LINKED COMPOSITION, PERSONAL CARE COMPOSITION INCLUDING THE SAME, AND METHODS OF FORMATION**
VERNETZTE ZUSAMMENSETZUNG, KÖRPERPFLEGEZUSAMMENSETZUNG DAMIT UND VERFAHREN ZUR HERSTELLUNG
COMPOSITION RÉTICULÉE, COMPOSITION DE SOINS PERSONNELS LES COMPRENANT, ET PROCÉDÉS DE FORMATION

(30) Priority: 30.04.2018 US 201862664654 P
(43) Date of publication of application: 10.03.2021
(73) Proprietor: Dow Silicones Corporation, Midland, MI 48686-0994 (US); Dow Global Technologies, LLC, Midland, MI 48674 (US)
(72) Inventor: BAUMGARTNER, Ryan, Midland, Michigan 48686-0994 (US); HALLER, Roxanne Renee, Midland, Michigan 48686-0994 (US); JELETIC, Matthew, Midland, Michigan 48686-0994 (US); LAITAR, David, Midland, MI 48674 (US); NGUYEN, Kimmai Thi, Midland, Michigan 48686-0994 (US); ZHANG, Fang, Midland, Michigan 48686-0994 (US); ZIMMERMAN, Kenneth Edward, Midland, Michigan 48686-0994 (US)
(74) Representative: Murgitroyd & Company
(86) International application number: PCT/US2019/029961
(87) International publication number: WO 2019/213112

(56) References cited:
- WO-A1-98/33787
- WO-A1-2015/066161
- WO-A1-2015/066165

## Description

The present invention generally relates to a cross-linked composition comprising the reaction product of siloxanes having anhydride groups and a polyol having hydroxyl groups reactive with the anhydride groups. In addition, the present invention generally relates to a method of forming the cross-linked composition, a personal care composition comprising the cross-linked composition, and a method of forming the personal care composition.

Silicone elastomer gels/blends have been used extensively to enhance the aesthetics of personal care formulations for skincare and healthcare by providing a unique sensory profile upon application. For example, such gels/blends can provide sensory characteristics such as a velvety, silky or powdery feel. In addition, such gels/blends are also valued for providing rheology modification to personal care (e.g. skin, sun, cosmetic) and healthcare formulations.

Most silicone elastomer gels are obtained by a cross-linking hydrosilylation reaction of a SiH functional polysiloxane with another polysiloxane containing an unsaturated hydrocarbon substituent, such as a vinyl functional polysiloxane, or by cross-linking a SiH functional polysiloxane with a hydrocarbon diene or with a terminally unsaturated polyoxyalkylene (e.g. PEG/PPG). These silicone elastomer gels are compatible with mostly non-polar organic solvents. Unfortunately, such silicone elastomer gels have limited versatility in formulations with polar solvents such as hydrocarbon oils, ester oils and plant based oils.

While various silicones have been proposed, they suffer from one or more deficiencies. For example, some silicones are malodorous, while other silicones utilize reactants that are cost prohibitive. In addition, some silicones or components thereof suffer from stability issues, which can lead to gelling or reduced shelf life. In view of the foregoing, there remains an opportunity to provide cost effective silicones with little to no odor and increased formulation versatility, as well as to provide silicones having excellent aesthetic and rheological properties. There also remains an opportunity to provide improved personal care compositions, such as improved cosmetic compositions, and improved methods of forming such compositions.

Cross-linked compositions comprising the reaction product of: a first siloxane having at least one pendant anhydride group; a second siloxane having at least one pendant anhydride group; and a reactant comprising an organic polyol having at least two hydroxyl groups reactive with the pendant anhydride groups of said first and second siloxanes are known from WO 2015/066161.

### BRIEF SUMMARY OF THE INVENTION

A cross-linked composition is provided. In various embodiments, the cross-linked composition comprises the reaction product of: a first siloxane having at least one pendant polycyclic anhydride group; a second siloxane having at least one pendant polycyclic anhydride group; and a polyol having at least two hydroxyl groups reactive with the pendant polycyclic anhydride groups of the first and second siloxanes.

In certain embodiments, the cross-linked composition comprises the reaction product of: a first siloxane having at least one pendant anhydride group; a second siloxane having at least one pendant anhydride group; and a polyol having at least two hydroxyl groups reactive with the pendant anhydride groups of the first and second siloxanes. In these embodiments, at least one of the pendant anhydride groups comprises an unsaturated bond.

In various embodiments, the cross-linked composition comprises the following general formula (I):

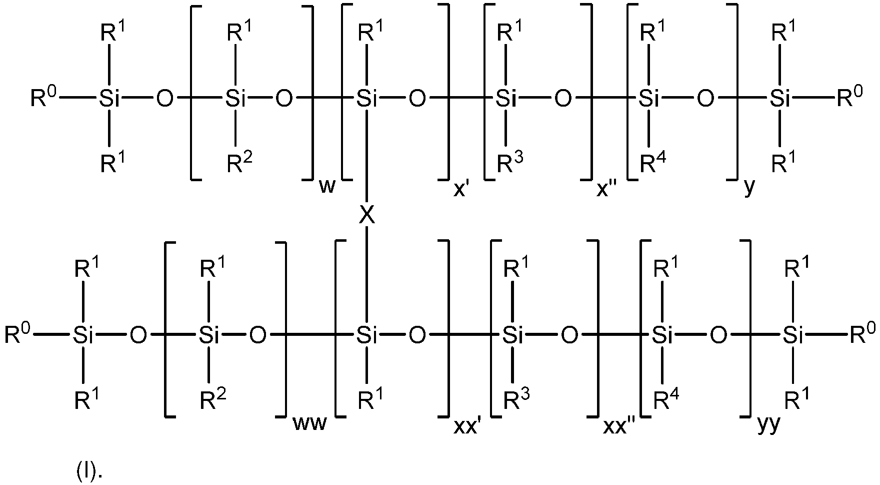

In formula (I) above, each of R⁰, R¹, and R² is an independently selected substituted or unsubstituted hydrocarbyl group. Each R⁴ is an independently selected substituted or unsubstituted hydrocarbyl group. Each of subscripts w and ww is an independently selected integer of from 0 to 1,000. Each of subscripts x' and xx' is an independently selected integer ≥1. Each of subscripts x" and xx" is an independently selected integer ≥0. The sum of subscripts x'+x" is an integer of from 1 to 100. The sum of subscripts xx'+xx" is an integer of from 1 to 100. Each of subscripts y and yy is an independently selected integer of from 0 to 1,000.

In certain embodiments, each R³ comprises or is a polycyclic anhydride group of the following general formula (A1):

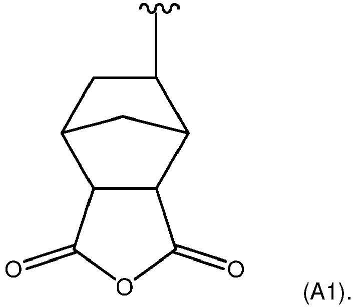

In formula (I) above for these embodiments, "X" comprises or is of the following general formulae (i-a), (i-b) or (i-c):

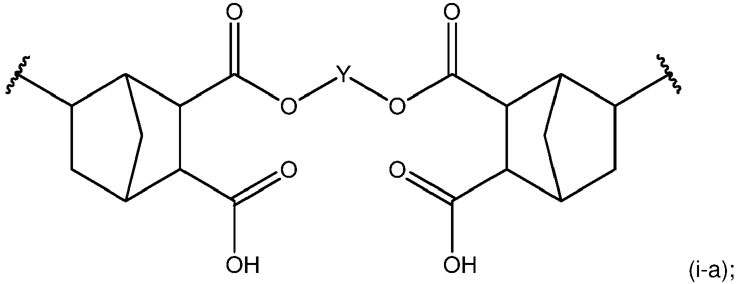

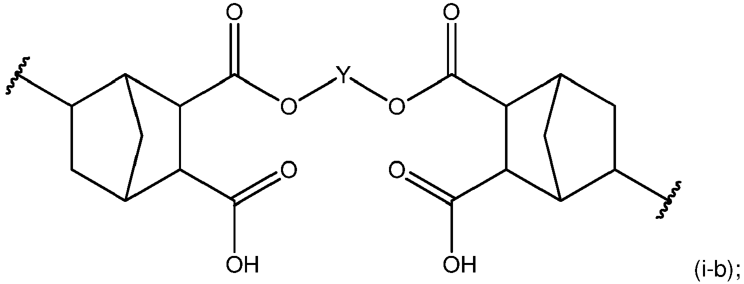

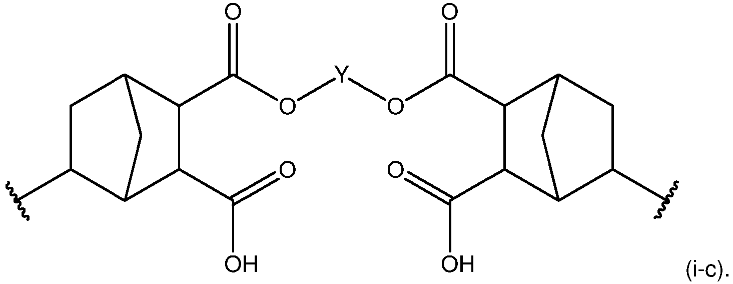

In other embodiments, each R³ comprises or is a polycyclic anhydride group of the following general formula (A2): 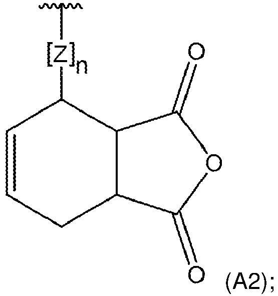 where Z is a divalent linking group, and n is 0 or an integer greater than zero.

In formula (I) above for these other embodiments, "X" comprises or is of the following general formulae (ii-a), (ii-b) or (ii-c):

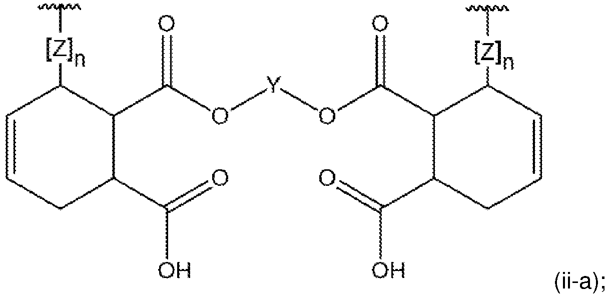

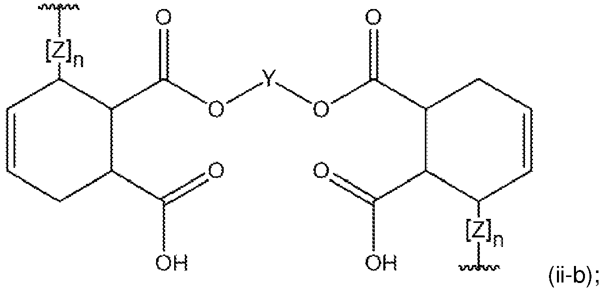

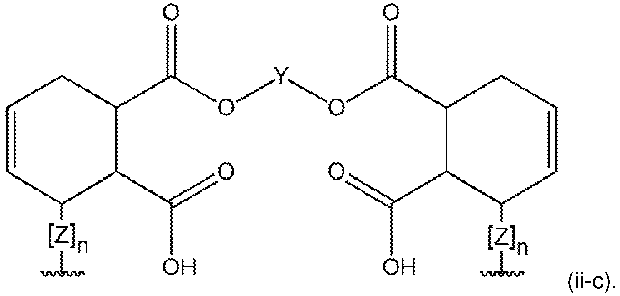

In formula (ii) above, Z is a divalent linking group, and n is 0 or an integer greater than zero. In formulae (i) or (ii) above, "Y" is a divalent group formed from a polyol having at least two hydroxyl groups.

A method of forming a cross-linked composition is also provided. The method comprises providing an organohydrogenpolysiloxane of the following general formula (B):

R⁰R¹₂Si-O-[SiR¹R²-O-]_{w}[SiHR¹-O-]ₓ[SiR¹R⁹-O-]_{y}SiR¹₂R⁰ (B).

In formula (B) above, each R⁰ is an independently selected substituted or unsubstituted hydrocarbyl group or a hydrogen (H). Each of R¹ and R² is an independently selected substituted or unsubstituted hydrocarbyl group. R⁹ is a substituted or unsubstituted hydrocarbyl group or a hydrogen. Subscript w is an integer of from 0 to 1,000. Subscript x is an integer of from 1 to 100. Subscript y is an integer of from 0 to 1,000.

The method further comprises providing an alkenyl functional polycyclic anhydride. The method further comprises providing a polyol having at least two hydroxyl groups. Optionally, the method further comprises providing a linear alkene having 2 to 20 carbon atoms.

The method further comprises combining the organohydrogenpolysiloxane and the alkenyl functional polycyclic anhydride, and optionally the linear alkene, in the presence of a hydrosilylation catalyst to form a reaction intermediate. The reaction intermediate comprises siloxanes having at least one pendant polycyclic anhydride group. The method further comprises combining the siloxanes and the polyol to form the cross-linked composition. In various embodiments, the cross-linked composition is as described above.

A personal care composition is also provided. The personal care composition comprises a cross-linked composition having at least two carboxyl groups. In various embodiments, the cross-linked composition is as described above. The personal care composition further comprises a least one cosmetic component. Optionally, the personal care composition further comprises a cosmetically acceptable medium. For example, the cosmetic component(s) can be in the cosmetically acceptable medium.

A polycyclic anhydride composition is also provided. The polycyclic anhydride composition comprises the reaction product of: a triene compound; and maleic anhydride. The triene compound is of the following general formula:

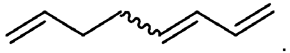

A polyorganosiloxane composition is also provided. The polyorganosiloxane composition comprises the reaction product of: a polycyclic anhydride compound; and an organosilicon compound having at least one silicon-bonded hydrogen atom. The polycyclic anhydride compound is of the following general formula:

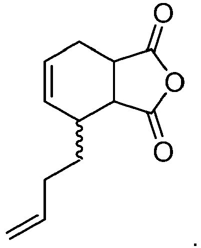

### DETAILED DESCRIPTION OF THE INVENTION

In various embodiments, the cross-linked composition may be referred to herein as a cross-linked siloxane composition or simply as an elastomer. The elastomer may be referred to as a siloxane-based elastomer or a polysiloxane-based elastomer. In various embodiments, the personal care composition may be referred to herein as a cosmetic composition.

### Cross-linked Composition

The first and second siloxanes are referred to collectively as "the siloxanes". In certain embodiments, the cross-linked composition consists essentially of the reaction product of the siloxanes and polyol. In further embodiments, the cross-linked composition consists of the reaction product of the siloxanes and polyol. In certain embodiments, the cross-linked composition can include one or more siloxanes in addition to, and different from, the first and second siloxanes (and different from a third siloxane described below).

In a first general embodiment of the disclosure ("first embodiment"), the polyol comprises an organic polyol. In the first embodiment, the polyol may also consist essentially of, optionally consist of, the organic polyol. In a second general embodiment of the disclosure ("second embodiment"), the polyol comprises a third siloxane having at least two hydroxyl groups. In the third embodiment, the polyol may also consist essentially of, optionally consist of the third siloxane. The third siloxane may also be referred to as a hydroxyl functional siloxane or a hydroxyl functional polysiloxane. In further embodiments, the polyol comprises a combination of two or more of the aforementioned polyols. For example, the polyol can comprise one or more organic polyols and/or one or more third siloxanes.

In certain embodiments, the siloxanes used to form the elastomer consist of siloxane bonds (Si-O-Si) within each of their backbones. Alternatively, each of the siloxanes may include siloxane bonds separated by one or more bivalent groups, e.g. a -CH₂- linking group.

Further examples of suitable bivalent groups include polyether groups, e.g. a -CH₂CH₂O-linking group (i.e., an EO group), a -CH(CH₃)CH₂O- linking group (i.e., a PO group), etc. Combinations of different bivalent groups may be present within each of their backbones. Each of the bivalent groups may be singular or repeated, e.g. 2 times, 5 times, 10 times, >10 times, etc. In certain embodiments, the first and second siloxanes are free of polyether groups.

In various embodiments, each of the siloxanes comprise at least one [SiR₂-O-] unit ("D" or R₂SiO_{2/2} units). In further embodiments, each of the siloxanes has repeating D units, which generally constitute linear portions of the siloxanes. The amount of repeating D units may also be referred to as the degree-of-polymerization ("DP") of the siloxane backbone. In yet further embodiments, each of the siloxanes has terminal R₃SiO_{1/2} ("M") units. In these embodiments, R is an independently selected substituted or unsubstituted hydrocarbyl group. By "substituted," it is meant that one or more hydrogen atoms of the hydrocarbon may be replaced with atoms other than hydrogen (e.g. a halogen atom), or a carbon atom within the chain of R may be replaced with an atom other than carbon, i.e., R may include one or more heteroatoms within the chain, such as oxygen, sulfur, nitrogen, etc.

In certain embodiments, each of the siloxanes described herein may optionally be branched, partially branched, and/or may include a resinous portion having a three-dimensional networked structure. In such embodiments, the respective siloxane may further comprise RSiO_{3/2} ("T") units and/or SiO_{4/2} ("Q") units. Branching of the siloxane, or the resinous portion, if present, can be attributable to the presence of T units and/or Q units. Branching may also be attributable to side groups of one or more D units. In various embodiments, the siloxanes are free of T units, Q units, or both T and Q units. Where more than one siloxane is described, the siloxanes can be the same or different, e.g. one is linear and one is branched, both are branched, both are linear, etc.

In certain embodiments, the cross-linked composition can be formed with a supplemental cross-linker in addition to the polyol. Examples of suitable supplemental cross-linkers include other polyols, polyamines, polyepoxides, and combinations thereof. Suitable supplemental cross-linkers, as well as other optional components that can be used to form, and/or be used in combination with the cross-linked composition are described in U.S. Pat. Nos. 5,444,139 and 8,026,330; and U.S. Pat. App. Pub. No. 2012/0040931. Further suitable supplemental cross-linkers and siloxanes that can be used to form, and/or be used in combination with the cross-linked composition, are described in U.S. Pat. App. Pub. Nos. 2016/0194456, 2016/0199286, 2016/0200876, and 2017/0065514; and U.S. Pat. Nos. 9,822,221 and 9,714323. Combinations of cross-linkers, supplemental cross-linkers, (functional and/or non-functional) resins, and/or siloxanes, can be utilized.

Referring back to general formula (I) illustrated above, suitable hydrocarbyl groups include alkyl, aryl, alkenyl, alkaryl, and aralkyl, groups. In various embodiments, each of R⁰, R¹, and R² is an independently selected alkyl group. Suitable alkyl groups can be linear, branched, or cyclic. If present as R⁰, R¹, and/or R², the alkyl group generally has from 1 to 20, 1 to 15, 1 to 10, 1 to 6, 1 to 4, 1, or 2, carbon atoms, or any number of carbon atoms in between. Specific examples of suitable alkyl groups include methyl groups, ethyl groups, propyl groups, butyl groups, pentyl groups, etc. In certain embodiments, at least one of R⁰, R¹, and R² is a methyl group (i.e., -CH₃). In further embodiments, each of R⁰, R¹, and R² is a methyl group.

In various embodiments, each R⁴ is an independently selected alkyl group, aryl group, or polyether group. In certain embodiments, each R⁴ is independently R¹ or an alkyl group having from 2 to 20 carbon atoms. In further embodiments, each R⁴ is independently an alkyl group having from 4 to 10 carbon atoms, optionally 6 to 8 carbon atoms.

If R⁴ is a polyether group, the polyether group generally terminates with an -OR' group. R' may be an alkyl group or aryl group, and there may be from 2 to 49, 2 to 24, 2 to 9, 2 to 4, or 2, ether linkages, or any number of ether linkages in between. In certain embodiments, R⁴ is an independently selected alkyl group having from 2 to 20, 2 to 15, 2 to 10, 2 to 8, 4 to 6, 5, or 6, carbon atoms, or any number of carbon atoms in between. Without being bound or limited by any particular theory, it is thought that the organic compatibility of the elastomer, e.g. in a solvent, can be enhanced by having a long chain alkyl group on one or both of the first and second siloxane backbones, e.g. as R⁴.

In various embodiments, each of w and ww is an independently selected integer of from 1 to 300. In certain embodiments, each of w and ww is an independently selected integer of from 50 to 200. In further embodiments, each of w and ww is an independently selected integer of from 75 to 125.

In various embodiments, each of x' and xx' is an independently selected integer ≥2. In certain embodiments, each of x' and xx' is an independently selected integer of from 2 to 20. In various embodiments, each of x" and xx" is an independently selected integer ≥1. In certain embodiments, each of x" and xx" is an independently selected integer of from 1 to 20.

In various embodiments, the sum of x'+x" is equal to subscript x as used herein. In certain embodiments, the sum of x'+x" is an integer of from 2 to 75. In further embodiments, the sum of x'+x" is an integer of from 2 to 50. In various embodiments, the sum of xx'+xx" is equal to subscript xx as used herein. In certain embodiments, the sum of xx'+xx" is an integer of from 1 to 75. In further embodiments, the sum of xx'+xx" is an integer of from 1 to 50.

In various embodiments, each of y and yy is an independently selected integer of from 1 to 300. In certain embodiments, each of y and yy is an independently selected integer of from 1 to 200. In further embodiments, each of y and yy is an independently selected integer of from 1 to 20.

The groups represented by subscripts w, ww, x, xx, y, and yy, e.g. the groups having square brackets in formulas illustrated herein, may be present in any order within the respective siloxane backbone, including a different order than that which is represented above and throughout this disclosure. Moreover, these groups may be present in randomized or block form.

Referring to the method of forming a cross-linked composition and the organohydrogenpolysiloxane of general formula (B) illustrated above, each of R¹ and R² is as described above. In addition, each of subscripts w, x (e.g., the sum of x'+x"), and y is as described above.

In certain embodiments, at least one or each R⁰ is an independently selected substituted or unsubstituted hydrocarbyl group. In further embodiments, at least one or each R⁰ is an independently selected alkyl group. Suitable alkyl groups are as described above for R¹. In other embodiments, at least one or each R⁰ is a hydrogen. In these embodiments, the hydrogen may be used as a reactive site for attaching at least one terminal polycyclic anhydride group. Other groups may also be attached. For example, the hydrogen may be reacted with and a linear alkene.

In certain embodiments, R⁹ is a substituted or unsubstituted hydrocarbyl group. In these embodiments, R⁹ can be R⁴, with R⁴ as described above. In other embodiments, R⁹ is a hydrogen. In these embodiments, the hydrogen may be used as a reactive site for attaching the group that becomes or is R⁴.

In various embodiments, the cross-linked composition can be prepared by:
(1) subjecting the organohydrogenpolysiloxane and the alkenyl functional polycyclic anhydride to an addition reaction to form the siloxanes; and
(2) subjecting the siloxanes obtained in the step (1) to a ring-opening reaction by combining them with the polyol thereby forming the cross-linked composition.

Suitable examples of the alkenyl functional polycyclic anhydride include those of the following general formulae (A-1) and (A-2):

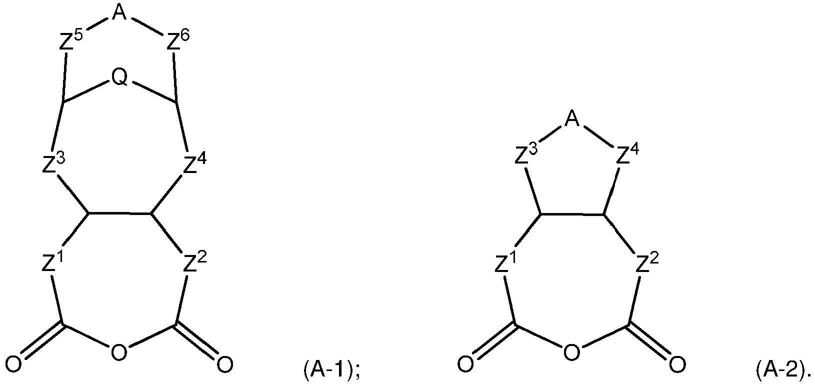

In formulae (A-1) and (A-2) above, "A" is one of the following two groups:

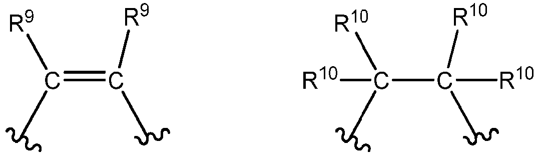

In these groups, each R⁹ is an independently selected substituted or unsubstituted hydrocarbyl group or a hydrogen. In various embodiments, each R⁹ is a hydrogen. Each R¹⁰ is an independently selected substituted or unsubstituted hydrocarbyl group or a hydrogen, provided at least one of R¹⁰ has alkenyl functionality.

"Q" is an oxygen (O) or Z⁷. In various embodiments, Q is Z⁷, where Z⁷ is (CR⁹₂)ₑ. Here, each R⁹ is an independently selected substituted or unsubstituted hydrocarbyl group or a hydrogen. In various embodiments, each R⁹ is a hydrogen. Subscript e is an integer of from 1 to 3, optionally 1 or 2. In certain embodiments, Q is (CH₂).

Each of Z¹, Z², Z³, Z⁴, Z⁵, and Z⁶ is an independently selected (CR⁹₂)_{f}, where R⁹ is an independently selected substituted or unsubstituted hydrocarbyl group or a hydrogen. In various embodiments, each R⁹ is a hydrogen. Subscript f is an integer of from 0 to 3, optionally 0 to 2, or 0 or 1. In certain embodiments, each of Z¹, Z², Z³, Z⁴, Z⁵, and Z⁶ is (CH₂)_{f}. In further embodiments, f is 0 or 1. In embodiments where f is 0, the respective Z group is not present as an intermediate divalent group.

In various embodiments, the R³ group is attributable to the alkenyl functional polycyclic anhydride of formula (A-1). In further embodiments, the alkenyl functional polycyclic anhydride comprises carbic anhydride:

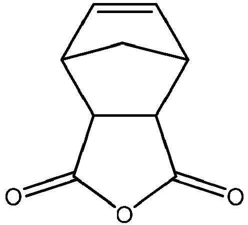

Carbic anhydride may also be referred to as 5-Norbornene-2,3-dicarboxylic anhydride. These and other alkenyl functional polycyclic anhydrides, components, and methods of formation suitable for this disclosure are described in in U.S. Pat. Nos. 4,381,396 and 5,015,700

In various embodiments, the R³ group is attributable to the alkenyl functional polycyclic anhydride of formula (A-2). In certain embodiments, the R³ group is attributable to the alkenyl functional polycyclic anhydride of formula (A-2-i); and in further embodiments, the alkenyl functional polycyclic anhydride comprises the general formula (A-2-ii):

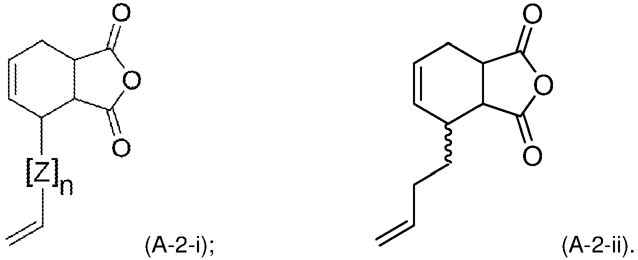

In formula (A-2-i) above, Z is a divalent group (or spacer) and n is 0 or an integer greater than zero. For example, Z can be a hydrocarbylene group having from 1 to 20 carbon atom(s), e.g. -CH₂-. Other options for Z are described further below. Subscript n can be, for example, 0 or range from 1 to 20, from 1 to 10, or from 2 to 5, or be 1, 2, 3, 4 or 5. Each of Z and n can also apply to formulae (I), (A2), (ii) and/or (iii) herein.

The alkenyl functional polycyclic anhydride of formula (A-2-ii) may be referred to as 3-(but-3-en-1-yl)-1,2,3,6-tetrahydrophthalic anhydride or as "BTHPA".

In various embodiments, the R³ group is attributable to carbic anhydride. In other embodiments, the R³ group is attributable to BTHPA. In certain embodiments, the R³ groups are attributable to both carbic anhydride and BTHPA. For example, a combination of different alkenyl functional polycyclic anhydrides can be used to form one or more of the siloxanes. As a further example, one or more of the siloxanes can comprise a combination of the polycyclic anhydride groups attributable to formulae (A-1) and (A-2), and such combinations can be the same of different. For example, one siloxane may have more polycyclic anhydride groups attributable to formula (A-1) relative to formula (A-2), whereas another siloxane may have more polycyclic anhydride groups attributable to formula (A-2) relative to formula (A-1).

One of skill in the art will appreciate that using combinations of different siloxanes can provide various cross-linked compositions. For example, cross-linking reaction of siloxanes having different polycyclic anhydride groups can provide the cross-linked composition of formula (I) where X is of the following general formula (iii-a):

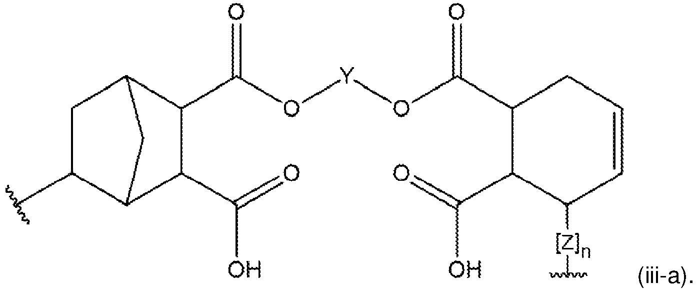

One of skill in the art will also appreciate that while not shown, other isomers of formula (iii) are also possible (e.g., as like illustrated above for formulae (i) and (ii)). Each of Y, Z and n is as defined above.

Optionally, in embodiments where the first and/or second siloxane has side chains as one or more of the R⁴ groups, step (1) can also include providing at least one component that is reactive with hydrogen atoms at the site(s) where R⁴ is to be bonded, e.g. at R⁹. Various types of components can be utilized to provide R⁴.

Typically, the component will have an unsaturated bond. Examples of suitable components include alkenes, such as those having from 2 to 20 carbon atoms; aldehydes; ketones; and combinations thereof. Specific examples of suitable alkenes include, but are not limited to, ethene, propene, 1-butene, 1-pentene, 1-hexene, 1-hexadecene, and combinations thereof. Components that impart a polyether group can be used. Vinyl terminated siloxanes may also be used as the component. Combinations of different components can be utilized to impart the R⁴ groups. If utilized, the component can be introduced prior to, after, or simultaneously with, the alkenyl functional polycyclic anhydride. The amount of each can be tailored to impart the cross-linked composition with various levels of each of the R³ and R⁴ groups. R⁴ may already be present on the organohydrogenpolysiloxane such that this optional step is not required.

The addition reaction in step (1) may also be referred to as a hydrosilylation reaction. The addition reaction in step (1) may be performed in the presence of catalyst, such as a platinum catalyst or a rhodium catalyst.

Suitable catalysts comprise platinum group metals (sometimes referred to as platinum metals) i.e. platinum, ruthenium, rhodium, palladium, osmium and iridium or complexes or compounds of a platinum group metal. In various embodiments, the catalyst is selected from the group of platinum compounds or complexes including chloroplatinic acid, platinum acetylacetonate, complexes of platinous halides with unsaturated compounds, for example, ethylene, propylene, organovinylsiloxanes and styrene, hexamethyldiplatinum, PtCl₂, PtCl₃ and Pt(CN)₃. In certain embodiments, the catalyst comprises Karstedt's catalyst, a coordination complex of platinum and divinyltetramethyldisiloxane produced by reaction of chloroplatinic acid and divinyltetramethyldisiloxane. In other embodiments, the catalyst comprises a rhodium complex, e.g., RhCl₃(Bu₂S)₃. An amount of the catalyst to be used may be a catalytically effective amount, i.e., a catalytic amount, such as at most 50 ppm, optionally at most 20 ppm, as platinum metal or rhodium metal.

The addition reaction in step (1) may be performed in a solvent as needed. Various types of conventional solvents can be utilized, such as silicone solvents and/or organic solvents. A specific example of a suitable silicone solvent is 3-octylheptamethyltrisiloxane. Examples of suitable organic solvents include, but are not limited to, aromatic hydrocarbons such as toluene and xylene; aliphatic or alicyclic hydrocarbons such as isododecane, n-pentane, n-hexane, and cyclohexane; and halogenated hydrocarbons such as dichloromethane, chloroform, and carbon tetrachloride. Additional examples of suitable solvents are described as "carrier fluids" in U.S. Pat. App. Pub. No. 2010/0330011.

Reaction conditions for the addition reaction in step (1) are not limited to any particular ones. In certain embodiments, the addition reaction is performed under reflux for 1 to 10 hours. While step (1) is described above, the siloxanes may also be provided "as is", i.e., they need not be first formed via such an addition reaction step. Suitable siloxanes are available from Dow Silicones Corporation of Midland, Ml.

Optionally, rather than just cross-linking the anhydride groups of the siloxanes in step (2), one or more of the functional groups provided by the opened anhydride groups may be capped. Various types of capping components can be utilized. Typically, the capping component will have at least one functional group, e.g. a hydroxyl group, an amine group, etc. Examples of suitable capping components include branched and unbranched aliphatic, cycloaliphatic, and aromatic monols and/or monoamines. Various types of capping components can be utilized, such as those having from 1 to 20 carbon atoms. Specific examples of suitable monols include, but are not limited to, methanol, ethanol, propanol, isopropanol, butanol, sec-butanol, isobutanol, *tert*-butanol*,* and the various isomers of pentyl alcohol, hexyl alcohol, octyl alcohol (e.g. 2-ethylhexanol), nonyl alcohol, decyl alcohol (e.g. 2-propylheptanol), lauryl alcohol, myristyl alcohol, cetyl alcohol and of stearyl alcohol, as well as the fatty alcohols and wax alcohols which occur naturally or which can be obtained by the hydrogenation of naturally occurring carboxylic acids. Cyclohexanol and its homologues are examples of suitable cycloaliphatic alcohols. Further, aromatic hydroxyl compounds, such as phenol, cresol, thymol, carvacrol, benzyl alcohol and phenylethanol, can also be utilized. In certain embodiments, the capping component is selected from the group of aliphatic alcohols, such as methanol, ethanol, 2-propanol, butanol, and isododecane. Combinations of different capping components can be used. If utilized, the capping component can be introduced prior to, after, or simultaneously with, the polyol. The amount of each can be tailored to impart the cross-linked composition with various levels of cross-linking, capping, free anhydride groups, and/or free carboxyl groups. Capping is optional.

The ring-opening reaction in the step (2) may be performed in a solvent as needed. Examples of suitable solvents include those listed for step (1). To prevent undesirable side-reactions/reaction-products, the solvent should be inert with respect to the reactants/reaction-intermediates. For example, the solvent shouldn't have hydroxyl or amine functional groups. This is generally true for steps (1) and (2). The reaction conditions for the ring-opening reaction are not limited to any particular ones. In certain embodiments, the ring-opening reaction is performed at a temperature of from room temperature to a reflux temperature for 1 to 10 hours.

The first siloxane, second siloxane, and polyol (collectively "the reactants") can be reacted in various amounts to form the cross-linked composition. Based on the number of hydroxyl groups provided by the polyol, relative to the number of anhydride groups provided by the siloxanes, the reactants can be utilized in a 1:1 stoichiometric ratio. For example, one hydroxyl group can be present for every one of the anhydride groups present. Alternatively, the polyol can be utilized in a stoichiometric excess relative to the siloxanes. Conversely, the siloxanes can be utilized in a stoichiometric excess relative to the polyol. Such situations may also be referred to as over-indexing or under-indexing the ring-opening reaction, with an index of 1.0 (or 100) indicating that there is a stoichiometric amount of hydroxyl groups present to react with the amount of anhydride groups present (1:1). The index may be from 0.25 to 2.0, 0.5 to 1.5, 0.9 to 1.1, 0.95 to 1.05, or 1.0, or any number in between. Higher or lower indexes may also be utilized.

Based on the particular index utilized, various situations can arise. Specifically, the cross-linked composition can include various functional groups for subsequent reaction, including free carboxyl groups, and possibly even free anhydride groups and/or free hydroxyl groups, or combinations thereof. In various embodiments, the cross-linked composition does not include free hydroxyl groups. In certain embodiments, the cross-linked composition has a least two carboxyl groups. The disclosure is not limited to any particular subsequent reaction or use of such free functional groups. Various degrees of cross-linking can be present in the cross-linked composition based on the index utilized to form the cross-linked composition, from various degrees of partial cross-linking to full cross-linking.

The elastomer may also be referred to as a carboxy elastomer, a carboxy functional elastomer, or a carboxylic acid functional elastomer. In certain embodiments having free carboxyl groups (e.g. after a ring-opening reaction), the elastomer has a carboxyl equivalent of from 100 to 50,000, optionally 500 to 10,000, optionally 500 to 5,000, g/mol. For good handling property, the elastomer can have a viscosity of from 10 to 1,000,000, optionally 10 to 100,000, mm²/sec. Further, the elastomer can have a weight average molecular weight (reduced to polystyrene) of from 200 to 100,000, optionally 200 to 50,000.

In certain embodiments, a non-functionalized resin (i.e., one lacking reactive groups) is utilized with and/or in the elastomer. In these embodiments, at least a portion of the non-functionalized resin is trapped within the polymeric network during cure of the elastomer. Such non-functionalized resins can be useful for providing chemical and/or physical modifications to the elastomer.

Various non-limiting, more specific, embodiments of the elastomer (labeled as Elastomer 1 and Elastomer 2), in accordance with the general embodiments above, are described below. Combinations of the components for each of Elastomers 1 and 2 can be utilized to form further elastomers, and thus cosmetic compositions, not specifically elucidated herein.

### Elastomer 1 - Reaction Product of First and Second Siloxanes Having Pendant Polycyclic Anhydride Group(s) and Organic Polyol(s)

In a first embodiment, the elastomer ("Elastomer 1") comprises the reaction product of a reaction of the first siloxane, the second siloxane, and at least one organic polyol. In further embodiments, Elastomer 1 consists essentially of, optionally consists of, the reaction product of the first siloxane, second siloxane, and organic polyol(s). In certain embodiments, Elastomer 1 can include the further reaction product of one or more siloxanes in addition to, and different from, the first and second siloxanes.

Each of the first and second siloxanes has at least one, optionally at least two, pendant polycyclic anhydride group(s). The polycyclic anhydride groups may simply be referred to herein as anhydride groups. In various embodiments, each of the first and second siloxanes independently has from 1 to 25, 2 to 20, 3 to 15, 4 to 14, 5 to 12, 6 to 10, 7, 8, or 9, pendant polycyclic anhydride group(s). Pendant groups may also be referred to as side groups, and are different from terminal groups (sometimes referred to as end groups). In various embodiments, each of the first and second siloxanes is free of terminal anhydride groups. In certain embodiments, each of the anhydride groups is directly bonded to an intervening atom or linkage that is directly bonded to a silicon atom.

The anhydride groups are useful for reaction with the organic polyol, and can also impart additional functionality to Elastomer 1. It is thought that potential benefits provided by, or attributable to, the anhydride groups include, but are not limited to, film forming, substantivity, durability, pigment/particle suspension and/or modification, long lasting/wear, additional chemistry, actives (e.g. drug) or inactives (e.g. fragrance) delivery/release, hydrophilicity, reactivity, compatibility, polarity, and combinations thereof. In certain embodiments, the anhydride groups can provide free carboxyl groups, which can also provide benefits and/or be available for a subsequent, non-limiting reaction. For example, at least some of the anhydride groups are not reacting during the cross-linking reaction. Such anhydride groups may later react with water to provide carboxyl groups. In other embodiments, Elastomer 1 may have one or more free anhydride groups for a subsequent, non-limiting reaction.

The organic polyol has at least two hydroxyl groups reactive with the pendant polycyclic anhydride groups of the first and second siloxanes. Each of the hydroxyl groups can be pendant or terminal. In various embodiments, the organic polyol has two hydroxyl groups. In further embodiments, the organic polyol has two terminal hydroxyl groups and is free of pendant hydroxyl groups. Each of the hydroxyl groups can be directly bonded to a carbon atom, or to an intervening atom or linkage that is directly bonded to a carbon atom. Each of the hydroxyl groups can be primary, secondary, or tertiary, optionally primary or secondary, optionally primary. The hydroxyl groups are useful for reaction with the first and second siloxanes, and can also impart additional functionality to Elastomer 1. In various embodiments, all of the hydroxyl groups of the organic polyol cross-link with polycyclic anhydride groups of the first and second siloxanes to form linkages (e.g. ester cross-links). Some amount of anhydride and/or carboxyl groups can remain free depending on the amount of hydroxyl groups present during reaction to form Elastomer 1. Such free groups can be useful for subsequent reaction(s), e.g. with another component of the cross-linked composition, and/or can also interact with substrate surfaces, e.g. skin, leather, etc.

Each of the first and second siloxanes can be chemically (or physically) the same, such as two separate molecules of the same siloxane component (or type). For example, the first and second siloxanes can be provided together, such as in an "A-part" of a system for forming Elastomer 1 or provided separately, especially when they are different from each other. The organic polyol can be provided separate from the first and second siloxanes, such as in a "B-part" of a system for forming Elastomer 1.

In various embodiments, each of the first and second siloxanes comprise at least one [SiR¹R³-O] unit (i.e., D unit), optionally [SiR¹R³-O] units. In these embodiments, R¹ is an independently selected substituted or unsubstituted hydrocarbyl group. Examples of suitable groups represented by R¹ are as described above. R³ is also as described above, e.g. R³ is a polycyclic anhydride group of general formula (A1) or (A2).

In various embodiments, each of the first and second siloxanes is individually an organopolysiloxane of the following general formula (B1):

R⁰R¹₂Si-O-[SiR¹R²-O-]_{w}[SiR¹R³-O-]ₓ[SiR¹R⁴-O-]_{y}SiR¹₂R⁰ (B1)

.

In further embodiments, each of the first and second siloxanes is individually an organopolysiloxane of the following general formula (B2):

R¹₃Si-O-[SiR¹R²-O-]_{w}[SiR¹R³-O-]ₓ[SiR¹R⁴-O-]_{y}SiR¹₃ (B2).

In yet further embodiments, each of the first and second siloxanes is individually an organopolysiloxane of the following general formula (B3):

(CH₃)₃Si-O-[Si(CH₃)₂-O-]_{w}[Si(CH₃)R³-O-]ₓ[Si(CH₃)R⁴-O-]_{y}Si(CH₃)₃ (B3).

Each of R⁰, R¹, R², R³, and is R⁴ is as described above. In certain embodiments, R⁴ is either an alkyl group or a polyether group. Without being bound or limited to any particular theory, it is thought that the hydrophilic character of Elastomer 1 can be enhanced by having a polyether side chain (or chains) on one or both of the first and second siloxane backbones, e.g. as R⁴.

In various embodiments, w is an integer selected from zero (0) to 1,000, 0 to 950, 0 to 750, 0 to 500, 0 to 400, 1 to 350, 1 to 300, 25 to 250, 50 to 200, 50 to 150, 75 to 125, 90 to 110, 90 to 100, or 90 to 95, or any number/range in between. In certain embodiments, w≥1, w≥2, w≥3, w≥4, w≥5, w≥25, w≥50, or w≥75, and/or w≤1000, w≤750, w≤500, w≤250, w≤150, or w≤100. In specific embodiments, w is 90, 91, 92, 93, 94, or 95. In further embodiments, x is an integer selected from 1 to 100, 1 to 75, 1 to 50, 1 to 25, 1 to 20, 1 to 15, or 1 to 10, or any number/range in between. In certain embodiments, x≥1, x≥2, x≥3, x≥4, x≥5, x≥10, x≥15, or x≥20, and/or x≤50, x≤40, x≤30, x≤20, x≤15, or x≤10. In specific embodiments, x is 2, 3, 4, 5, 6, 7, 8, 9, or 10. In further embodiments, y is an integer selected from 0 to 1,000, 0 to 950, 0 to 750, 0 to 500, 0 to 400, 1 to 350, 1 to 300, 1 to 250, 1 to 200, 1 to 150, 1 to 100, 1 to 75, 1 to 50, 1 to 25, 1 to 20, 1 to 15, or 1 to 10, or any number/range in between. In certain embodiments, y≥1, y≥2, y≥3, y≥4, y≥5, y≥10, y≥15, or y≥20, and/or y≤50, y≤40, y≤30, y≤20, y≤15, or y≤10. In specific embodiments, y is 1, 2, 3, 4, 5, 6, 7, 8, 9, or 10.

In various embodiments, w and y are not simultaneously 0. Said another way, in these embodiments, each of the first and second siloxanes include at least one D unit associated with each of the x units and at least one of the w and y units in formulas (B) to (B3). In certain embodiments, the sum of w+x+y is from 25 to 1,500, 25 to 1,000, 25 to 900, 25 to 800, 25 to 700, 25 to 600, 25 to 500, 25 to 400, 25 to 300, 50 to 200, 75 to 150, 85 to 125, or 90 to 110, or any number/range in between. In these embodiments, x is as described above. Thus, each of the first and second siloxanes has at least one of the pendant polycyclic anhydride groups, and can have other side groups based on the presence of one or more D units associated with w and y.

The organic polyol can be any type of polyol provided it has at least two hydroxyl groups reactive with the pendant polycyclic anhydride groups of the first and second siloxanes. In this way, the organic polyol serves as a cross-linker between the first and second siloxanes to form Elastomer 1. Elastomer 1 may constitute just one moiety, or a plurality of moieties, imparted by the organic polyol depending on, for example, the number of pendant polycyclic anhydride groups attributable to the first and second siloxanes. In certain embodiments, Elastomer 1 can include the further reaction product of one or more polyols in addition to, and different from, the organic polyol.

By "organic", it is generally meant that the organic polyol contains predominantly carbon, e.g. a carbon backbone. While carbon is present, other atoms may also be present, such as oxygen atoms, hydrogen atoms, nitrogen atoms, etc. In many embodiments, the organic polyol is free of silicon, e.g. one or more silicon atoms.

In various embodiments, the organic polyol (hereafter "polyol") is a diol (i.e., the polyol has two hydroxyl groups). Examples of suitable diols include, but are not limited to, methylene glycol, ethylene glycol, diethylene glycol, propylene glycol, dipropylene glycol, butane diol, bisphenol A, 1,4-butanediol, 1,3-propanediol, 1,5-pentanediol, 1,7-heptanediol, 1,2-hexanediol, triethylene glycol, tripropylene glycol neopentyl glycol, and combinations thereof. In other embodiments, the polyol is a triol (i.e., the polyol has three hydroxyl groups).

In various embodiments, the polyol has the following general formula (II): HO-R⁵-OH. In certain embodiments, R⁵ comprises at least one of a hydrocarbylene, a heterohydrocarbylene, or an organoheterylene group, optionally is a hydrocarbylene group having from 1 to 20 carbon atoms. In further embodiments, R⁵ is selected from alkyl, cycloalkyl, alkyl cycloalkyl, aromatic, and alkylaromatic diradicals. Such diradicals generally have up to 50, up to 40, up to 30, up to 20, or up to 10, carbon atoms, or any number of carbon atoms between 1 and 50. The carbon chain which makes up the backbone of the polyol may be straight chained or branched. In certain embodiments, the polyol may have ether, thio, or amine linkages in its main chain. In specific embodiments, R⁵ is a hydrocarbylene group having from 1 to 10, 2 to 9, 3 to 8, 4 to 7, 5, or 6, carbon atom(s).

In certain embodiments, the polyol is a (poly)oxyalkylene compound. Suitable examples of such compounds include, but are not limited to, ethylene glycol, diethylene glycol, triethylene glycol, polyethylene glycol (e.g. having a molecular weight of 200 to 2,000), propylene glycol, dipropylene glycol, polypropylene glycol (e.g. having a molecular weight of 200 to 3,000), butylene glycol, dibutylene glycol, polybutylene glycol (e.g. having a molecular weight of 200 to 4,000), random copolymers and block copolymers of polyethylenepropylene glycol (e.g. having a molecular weight of 100 to 3,000), random copolymers and block copolymers of polyethylenebutylene glycol (e.g. having a molecular weight of 100 to 4,000), and combinations thereof.

In various embodiments, the polyol can comprise a polyester polyol, a polyether polyol, a polyether/ester polyol, or combinations thereof. Furthermore, the polyol may be selected from aliphatic polyols, cycloaliphatic polyols, aromatic polyols, heterocyclic polyols, and combinations thereof. Some examples of suitable polyols include, but are not limited to, glycol-initiated polyols, glycerine-initiated polyols, sucrose-initiated polyols, sucrose/glycerine-initiated polyols, trimethylolpropane-initiated polyols, and combinations thereof.

Suitable polyester polyols include hydroxyl-terminated reaction products of polyhydric alcohols, polyester polyols obtained by the polymerization of lactones, e.g. caprolactone, in conjunction with a polyol, and polyester polyols obtained by the polymerization of hydroxy carboxylic acids, e.g. hydroxy caproic acid. Polyesteramide polyols, polythioether polyols, polycarbonate polyols, polyacetal polyols, and polyolefin polyols may also be used.

Suitable polyether polyols include products obtained by the polymerization of a cyclic oxide, such as ethylene oxide (EO), propylene oxide (PO), butylene oxide (BO), and tetrahydrofuran in the presence of a polyfunctional initiator. Suitable initiator compounds contain a plurality of active hydrogen atoms, and include, but are not limited to, water, butanediol, ethylene glycol, propylene glycol, diethylene glycol, triethylene glycol, dipropylene glycol, ethanolamine, diethanolamine, triethanolamine, toluene diamine, diethyl toluene diamine, phenyl diamine, diphenylmethane diamine, ethylene diamine, cyclohexane diamine, cyclohexane dimethanol, resorcinol, bisphenol A, glycerol, trimethylolpropane, 1,2,6-hexanetriol, pentaerythritol, and combinations thereof. Some of these initiators may also be useful as the polyol itself. In specific embodiments, the polyol is a polyether diol. Combinations of different polyols can be utilized to form Elastomer 1.

Referring back to general formula (I) illustrated above, Elastomer 1 can comprise this formula. In formula (I), the upper and lower portions are attributable to the first and second siloxanes, which can be the same or different. The middle (or "X") portion of formula (I) is attributable to the polyol, as well as the polycyclic anhydride groups of the first and second siloxanes. The various R groups and subscripts are as described above.

Each of subscripts ww, xx, and yy can be the same as or different from each of w, x, and y, respectively. In various embodiments, ww and yy are not simultaneously 0. In certain embodiments, the sum of ww+xx+yy is from 25 to 1,500, 25 to 1,000, 25 to 900, 25 to 800, 25 to 700, 25 to 600, 25 to 500, 25 to 400, 25 to 300, 50 to 200, 75 to 150, 85 to 125, or 90 to 110, or any number/range in between. In these embodiments, xx is as described above.

Each Y is a divalent group, such as an organic divalent group, which is attributable to the polyol. During formation of Elastomer 1, the polyol had two hydroxyl groups, e.g. terminal hydroxyl groups, which reacted with pendant polycyclic anhydride groups of the first and second siloxanes to form linkages between the siloxanes and polyol. As shown in formula (I), Elastomer 1 has at least two carboxyl groups. In other embodiments (not shown), another molecule of the polyol has reacted between the two carboxyl groups to form another -Y- linkage (i.e., the two carboxyl groups in formula (I) are gone).

Y can be of any structure attributable to the polyol. In various embodiments, Y comprises at least one of a hydrocarbylene, heterohydrocarbylene, or organoheterylene group. In certain embodiments, Y is a hydrocarbylene group having from 1 to 50, 1 to 40, 1 to 20, 1 to 20, 1 to 10, 2 to 9, 3 to 8, 4 to 7, 5, or 6, carbon atom(s), or any number of carbon atoms in between. Further examples of suitable groups for Y are as described with the R⁵ groups of the polyol. In certain embodiments where the polyol is free of polyether groups, the Y is also free of polyether groups.

### Elastomer 2 - Reaction Product of First and Second Siloxanes Having Pendant Polycyclic Anhydride Group(s) and Third Siloxane(s) Having at Least Two Hydroxyl Groups

In a second embodiment, the elastomer ("Elastomer 2") comprises the reaction product of a reaction of the first siloxane, the second siloxane, and at least one third siloxane. In further embodiments, Elastomer 2 consists essentially of, optionally consists of, the reaction product of the first, second, and third siloxanes. In certain embodiments, Elastomer 2 can include the further reaction product of one or more siloxanes in addition to, and different from, the first, second, and third siloxanes. The first and second siloxanes are as described above, e.g. for Elastomer 1. The third siloxane can be provided separate from the first and second siloxanes, such as in a "B-part" of a system for forming Elastomer 2.

The third siloxane has at least two hydroxyl groups reactive with the pendant polycyclic anhydride groups of the first and second siloxanes. Each of the hydroxyl groups can be pendant or terminal. In various embodiments, the third siloxane has two hydroxyl groups. In further embodiments, the third siloxane has two terminal hydroxyl groups and is free of pendant hydroxyl groups. Each of the hydroxyl groups can be directly bonded to a silicon atom, or to an intervening atom or linkage that is directly bonded to a silicon atom. Alternatively, each of the hydroxyl groups can be directly bonded to a carbon atom, or to an intervening atom or linkage that is directly bonded to a carbon atom. Each of the hydroxyl groups can be primary, secondary, or tertiary, optionally primary or secondary, optionally primary. The hydroxyl groups are useful for reaction with the first and second siloxanes, and can also impart additional functionality to Elastomer 2. In various embodiments, all of the hydroxyl groups of the third siloxane cross-link with polycyclic anhydride groups of the first and second siloxanes to form linkages (e.g. ester cross-links). Some amount of anhydride and/or carboxyl groups can remain free depending on the amount of hydroxyl groups present during reaction to form Elastomer 2.

The third siloxane can be any type of siloxane provided it has at least two hydroxyl groups reactive with the pendant polycyclic anhydride groups of the first and second siloxanes. In this way, the third siloxane serves as a cross-linker between the first and second siloxanes to form Elastomer 2. Elastomer 2 may constitute just one moiety, or a plurality of moieties, imparted by the third siloxane depending on, for example, the number of pendant polycyclic anhydride groups attributable to the first and second siloxanes.

In various embodiments, the third siloxane comprises at least one D unit. In further embodiments, the third siloxane has repeating D units, which generally constitute linear portions of the siloxane. In various embodiments, the third siloxane is a carbinol siloxane or a carbinol polysiloxane.

In certain embodiments, the third siloxane is a polysiloxane of the following general formula (III):

HO-[Z]_{d}-[SiR⁶R⁷-O-]ₐ[SiR⁶R⁸-O-]_{b}[SiR⁶R⁷-O-]_{c}Si-[Z]_{d}-OH (III).

In formula (III) above, each of R⁶ and R⁷ is an independently selected substituted or unsubstituted hydrocarbyl group. Examples of suitable for groups for R⁶ and R⁷ are as described above, e.g. for R¹ and R⁴. R⁸ comprises an independently selected substituted or unsubstituted hydrocarbyl group or -[Z]_{d}-OH. In certain embodiments, R⁸ is R⁷. In other embodiments, where R⁸ is -[Z]_{d}-OH, the third siloxane includes at least one pendant hydroxyl group in addition to the two terminal hydroxyl groups.

Each Z is a divalent group, and in various embodiments can independently comprise at least one of a hydrocarbylene, a heterohydrocarbylene, or an organoheterylene group. In certain embodiments, Z is a hydrocarbylene group having from 1 to 20, 1 to 10, 1 to 5, 1 to 2, 1, or 2, carbon atom(s), or any number of carbon atoms in between. Further examples of suitable groups for Z are as described with the optional bivalent groups of the first and second siloxanes, e.g. a -CH₂- linking group, an EO group, a PO group, etc., or combinations thereof. In certain embodiments, the third siloxane is free of polyether groups.

In various embodiments, Z comprises at least one structural unit selected from the group consisting of: [(CH₂)ᵢ]ₖ; [(CH₂)ᵢO]ₖ; [(CH₂)ᵢ(CH)(CH₃)O]ₖ; [(CH₂)ᵢ(CH)(CH₂)ⱼ(CH₃)O]ₖ; [(CH)OH]ₖ; [(CH)(CH₂)ᵢOH]ₖ; [(CH₃)₂COH(CH₂)ᵢ]ₖ; [(CH₃)(CH₂)ᵢCOH(CH₂)ⱼ(CH₃)]ₖ; and combinations thereof. These units may be modified or rearranged to allow for proper orientation and bonding of the respective Z units in formula (III). In various embodiments, i is an integer selected from 1 to 100, 1 to 75, 1 to 50, 1 to 25, 1 to 10, 1 to 5, or 1, or any number/range in between; j is an integer selected from 1 to 100, 1 to 75, 1 to 50, 1 to 25, 1 to 10, 1 to 5, or 1, or any number/range in between; and k is an integer selected from 1 to 100, 1 to 75, 1 to 50, 1 to 25, 1 to 10, 1 to 5, or 1, or any number/range in between. Examples of Z include structural units (or moieties) attributable to use of 4-penten-1-ol, 7-octen-1-ol, glycerol monoallyl ether, allyl xylitol, trimethylolpropane monoallyl ether, xylitol, pentaerythritol, triglycerol, and combinations thereof. In certain embodiments, Z can include one or more pendant functional (e.g. hydroxyl) groups in addition to the terminal hydroxyl group attached thereto.

In various embodiments, a is an integer selected from 0 to 1,000, 1 to 950, 2 to 750, 3 to 500, 4 to 400, 5 to 300, 6 to 200, 7 to 100, 8 to 75, 9 to 50, 10 to 25, 11 to 20, or 12 to 15, or any number/range in between. In further embodiments, b is an integer selected from 1 to 1,000, 5 to 950, 10 to 750, 15 to 500, 25 to 400, 35 to 300, 45 to 200, or 50 to 100, or any number/range in between. In certain embodiments, b is an integer selected from 1 to 200 or any number/range in between. In further embodiments, c is an integer selected from 0 to 1,000, 1 to 950, 2 to 750, 3 to 500, 4 to 400, 5 to 300, 6 to 200, 7 to 100, 8 to 75, 9 to 50, 10 to 25, 11 to 20, or 12 to 15, or any number/range in between. In various embodiments, each d is independently 0 or 1. In specific embodiments, at least one d is 1, or both of d are 1.

The groups represented by subscripts a, b, and c, e.g. the groups having square brackets in formula (III), may be present in any order within the siloxane backbone, including a different order than that which is represented above and throughout this disclosure. Moreover, these groups may be present in randomized or block form.

In certain embodiments, the third siloxane is a silicone resin of the general formula R*ₛSiO_{(4-s)/2}. Typically, a silicone resin will have T and/or Q units, along with M units and, optionally, D units. R* can be an independently selected substituted hydrocarbyl group, unsubstituted hydrocarbyl group, or hydroxyl group, and s is from 0 to 3. Suitable R* groups are as described above for R¹, R², and R¹⁰. Various combinations of such groups can be present, provided the silicone resin has at least two hydroxyl groups per molecule (typically on M units). In these embodiments, the resin generally includes a combination of M, D, T, and/or Q units. In specific embodiments, the third siloxane is a MDT resin, a MT resin, a MDQ resin, a MQ resin, or a MDTQ resin. Each of the M, D, and T units can have differing R groups. The silicone resin can be of various molecular weights, including, but not limited to, a number average molecular weight of from 800 to 500,000, or any number/range in between.

Referring back to the general formula (I) illustrated above, Elastomer 2 can comprise this formula. In formula (I), the upper and lower portions are attributable to the first and second siloxanes, which can be the same or different. The middle (or "X") portion of formula (I) is attributable to the third polysiloxane, as well as the polycyclic anhydride groups of the first and second siloxanes. The various R groups and subscripts are as described above, e.g. for Elastomer 1.

Each Y is a divalent group, such as an organic divalent group, which is attributable to the third polysiloxane. During formation of Elastomer 2, the third polysiloxane had terminal hydroxyl groups, which reacted with pendant polycyclic anhydride groups of the first and second siloxanes to form linkages between the siloxanes and third polysiloxane. As shown in formula (I), Elastomer 2 has at least two carboxyl groups. In other embodiments (not shown), another molecule of the third polysiloxane has reacted between the two carboxyl groups to form another -Y- linkage (i.e., the two carboxyl groups in formula (I) are gone).

Y can be of any structure attributable to the third polysiloxane.

In various embodiments where the third siloxane is a polysiloxane, Y is of the following general formula (C):

-[Z]_{d}-[SiR⁶R⁷-O-]ₐ[SiR⁶R⁸-O-]_{b}[SiR⁶R⁷-O-]_{c}Si-[Z]_{d}- (C)

.

Each of the R groups and subscripts are as described above, although if R⁸ was -[Z]_{d}-OH, an alternate reaction product would be formed (not shown). In other embodiments where the third siloxane is a resin, Y is of the general formula R*ₛSiO_{(4-s)/2}. Each of R* and s is as described above.

### BTHPA, Fluid Composition and Methods of Preparation

As introduced above, the polycyclic anhydride composition comprises the reaction product of: the triene compound; and maleic anhydride. The reaction can occur in the presence of a solvent, such as an organic solvent (e.g. toluene). The triene compound is of the following general formula: 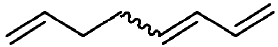 , which may be referred to as 1,3,7-octatriene. In general, the reaction product comprises BTHPA. It is believed that the stereochemistry of BTHPA is primarily the *endo* isomer; however, the *exo* isomer can also exist. As described above and further exemplified below the BTHPA can be used to form the cross-linked compositions of this disclosure. Alternatively, the BTHPA can be used to form functional siloxane fluids as also described below.

As introduced above, the polyorganosiloxane composition comprises the reaction product of: the polycyclic anhydride compound; and the organosilicon compound having at least one silicon-bonded hydrogen atom. In various embodiments, the polycyclic anhydride compound comprises or is BTHPA. In certain embodiments, the organosilicon compound comprises or is the organohydrogenpolysiloxane of general formula (B) described above. Formation of the reaction product can be conducted as like described above for the siloxanes, e.g. in the presence of a catalyst (e.g. platinum).

### Additional Cross-linked Compositions and Methods of Preparation

As introduced above, the cross-linked composition can comprise the reaction product of: a first siloxane having at least one pendant anhydride group; a second siloxane having at least one pendant anhydride group; and a polyol having at least two hydroxyl groups reactive with the pendant anhydride groups of the first and second siloxanes. In these embodiments, at least one of the pendant anhydride groups comprises an unsaturated bond.

These embodiments are similar to those described above, but alternate reactants are used relative to those described above (or in addition to those described above). For example, rather than using carbic anhydride or BTHPA, a compound such as the following can be used: 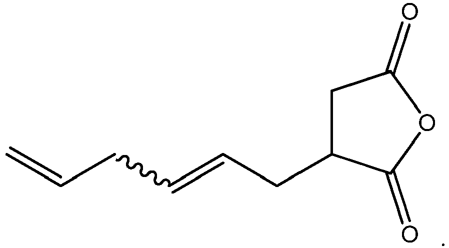 Alternatively, this compound can be used in addition to carbic anhydride and/or BTHPA. One of skill in the art can readily envision other similar structures, e.g. having different chain lengths and/or branching, provided they have an unsaturated bond that would generally be present after reaction to form the cross-linked composition.

The same type of method of forming a cross-linked composition as described above can be utilized here, but where the step of providing an alkenyl functional polycyclic anhydride is replaced by the step of providing an alkenyl functional anhydride (e.g. the compound above). In addition, the step of combining the organohydrogenpolysiloxane and the alkenyl functional polycyclic anhydride, and optionally the linear alkene, in the presence of a hydrosilylation catalyst is replaced with the step of combining the organohydrogenpolysiloxane and the alkenyl functional anhydride, and optionally the linear alkene, in the presence of a hydrosilylation catalyst to form a reaction intermediate comprising siloxanes having at least one pendant anhydride group comprising an unsaturated bond.

### Cosmetic Component

It is to be appreciated that certain components may be classified under different terms of art and just because a component is classified under such a term does not mean that they are limited to that function. As just one example, a component may be useful as a moisturizer but can also be useful for additional or alternate purposes, such as a diluent, an anti-aging active, an anti-bacterial agent, etc. The cosmetic component(s) may be present in the cosmetic composition in various amounts.

Cosmetic components are those components known to be used in cosmetic application. A wide review of such components may be found in the CTFA cosmetic component handbook. Exemplary cosmetic components are described in further detail below. Specific cosmetic components are described in U.S. Patent Appl. No. 62/664,654.

Cosmetic components include emollients, waxes, moisturizers, surface active materials such as surfactants or detergents or emulsifiers, thickeners, water phase stabilizing agents, pH controlling agents, preservatives and cosmetic biocides, sebum absorbants or sebum control agents, vegetable or botanical extracts, vitamins, proteins or amino-acids and their derivatives, pigments, colorants, fillers, silicone conditioning agents, cationic conditioning agents, hydrophobic conditioning agents, UV absorbers, sunscreen agents, antidandruff agents, antiperspirant agents, deodorant agents, skin protectants, hair dyes, nail care components, fragrances or perfume, antioxidants, oxidizing agents, reducing agents, propellant gases, and mixtures thereof.

Additional components that may be used in the cosmetic compositions include fatty alcohols, color care additives, anticellulites, pearlising agents, chelating agents, film formers, styling agents, ceramides, suspending agents, anti-aging actives, and others.

It is thought that the carboxyl groups of the elastomer may interact with certain cosmetic components, thus providing delivery of such component's to a subject's skin. Thus, in certain embodiments, the elastomer is reacted with the at least one cosmetic component. Examples of such interactions are thought to be hydrogen bonding, coordination, and/or chemical reaction between carboxyl groups of the elastomer and a functional group of the cosmetic component.

Further materials suitable for the personal care and health care are well known to the person skilled in the art and are described in many text books as well as other publications. For example, the CTFA Cosmetic Ingredient Dictionary, Second Edition (1977), includes a number of suitable cosmetic components for this disclosure.

### Cosmetically Acceptable Medium

A cosmetically acceptable medium is meant to designate a medium particularly suitable for applying a composition of the disclosure on keratin materials. The cosmetically acceptable medium is generally adapted to the nature of the support on which the cosmetic composition should be applied as well as to the aspect under which the cosmetic composition should be conditioned and includes water, solvents, diluents, or mixtures and emulsions thereof. When utilized, the cosmetically acceptable medium can be present in an amount ranging from 0.1% to 99.9% weight percent based upon the total weight of the cosmetic composition.

### Diluent

In various embodiments, a diluent is used. This is not to say that the elastomer requires the diluent, but certain end applications may find use of the diluent helpful. Use of the diluent is optional. In embodiments utilizing the diluent, the combination of the elastomer and diluent may be referred to as an elastomer composition or a blend.

In various embodiments, the diluent is selected from the group of silicones, organic oils, organic solvents, and combinations thereof. In certain embodiments, the diluent is selected form the group of silicones. Suitable diluents which can be used to form or in combination with the cross-linked composition are described in U.S. Pat. No. 6,200,581 and U.S. Pat. App. Pub. No. 2004/0044121. Specific examples of diluents are described in U.S. Patent Appl. No. 62/664,654.

In various embodiments, the elastomer and diluent can be combined under a shear force to form a base composition suitable in use to form cosmetic compositions. For example, elastomers containing 5 to 98 weight percent of the diluent are stable and form uniform pastes with a wide viscosity range. Any type of mixing and shearing equipment may be used to form the base composition, such as a batch mixer, planetary mixer, single or multiple screw extruder, dynamic or static mixer, colloid mill, homogenizer, sonolator, or a combination thereof.

### Stabilizing Additive

In various embodiments, a stabilizing additive is used for stabilizing the elastomer. This is not to say that the elastomer requires stabilization, but certain end applications may find use of the stabilizing additive helpful. Use of the stabilizing additive is optional. Suitable types of stabilizing additives and amounts thereof are described in U.S. Patent Appl. No. 62/664,654.

As introduced above, carboxy-functionality of the elastomers is useful for improving substantivity of the elastomers on keratinous substrates. However, inter and intra- molecular interactions between polar moieties in the material structure of the elastomers, seemingly causes issues for material handling and formulation homogeneity in the form of structuring or thickening. To decrease structuring in carboxy-functional elastomers prior to use, mixing (shear thinning) can disrupt the interactions temporarily and allow for better introduction into a formulation. Introduction of polar solvents (e.g. water, EtOH, etc.) can also disrupt structuring; however, these materials limit formulation flexibility, especially in anhydrous formulations.

### Cosmetic Composition and Methods of Preparation

Cosmetic compositions include those compositions which are intended to be placed in contact with the external parts of the human body (skin (epidermis), hair system, nails, mucosa, etc., also referred to as "keratinous substrates") or with the teeth and the mucous membranes of the oral cavity with a view exclusively or mainly to cleaning them, perfuming them, changing their appearance, protecting them, keeping them in good condition or correcting body odors. In some instances, cosmetic compositions may also include health care compositions. Cosmetic applications, and in some instances health care applications, include skin care, sun care, hair care, or nail care applications.

The amount of the elastomer in the cosmetic composition may vary. The general level of the elastomer in the cosmetic compositions may vary from about 0.1% to about 95% by weight, optionally from about 0.2% to about 50%, optionally from about 0.5% to about 25%, relative to the total weight of the cosmetic composition. The cosmetic component is present at a level of from about 0.01% to about 99.99% by weight, relative to the total weight (or 100 parts by weight) of the cosmetic composition. The cosmetic component may be a combination or mixture of cosmetic components as listed above.

In various embodiments, the cosmetic composition may be prepared by a process comprising the steps of mixing the elastomer (and optionally, the diluent and/or the stabilizing additive) according to any embodiment described above and at least one cosmetic component optionally in the presence of a cosmetically acceptable medium. In various embodiments, the elastomer and the diluent and/or the stabilizing additive are first combined to form a blend or a mixture. In certain embodiments, these are the only two or three components of the blend/mixture. It is thought that the stabilizing additive stabilizes the elastomer and generally prevents the elastomer from structuring and/or de-structures (i.e., thins) the elastomer if already structured or beginning to structure (i.e., thicken). This is useful for ease of formulation and handling. In further embodiments, the blend/mixture and the at least one cosmetic component are combined to form the cosmetic composition, optionally in the presence of the cosmetically acceptable medium. In these embodiments, the combining steps are generally separate.

The cosmetic compositions may be prepared by mixing the elastomer (and optionally, the stabilizing additive) in the aqueous phase with the appropriate phase components, or in the oil phase with the appropriate phase components, and optionally provide for a second phase, and mix both phases together, optionally under heating.

The process may be conducted at temperatures ranging of from 15 to 90°C, optionally of from 20 to 60°C, optionally at room temperature (at or about 25°C), using simple propeller mixers, counter-rotating mixers, or homogenizing mixers. In general, no special equipment or processing conditions are required. Depending on the type of composition prepared, the method of preparation will be different, but such methods are understood by those skilled in the art.

The cosmetic compositions may be in the form of a cream, a gel, a powder (free flowing powder or pressed), a paste, a solid, freely pourable liquid, an aerosol. The cosmetic compositions may be in the form of monophasic systems, biphasic or alternate multi phasic systems; emulsions, e.g. oil-in-water, water-in-oil, silicone-in-water, water-in-silicone; multiple emulsions, e.g. oil-in-water-in-oil, polyol-in-silicone-in-water, oil-in-water-in-silicone.

Skin care compositions include shower gels, soaps, hydrogels, creams, lotions and balms; antiperspirants; deodorants such as sticks, soft solid, roll on, aerosol, and pumpsprays; skin creams; skin care lotions; moisturizers; facial treatments such as wrinkle control or diminishment treatments; anti-aging compositions; exfoliates; body and facial cleansers; bath oils; perfumes; colognes; sachets; sunscreens; mousses; patches; pre-shave and after-shave lotions; shaving soaps; shaving lathers; depilatories; make-ups; color cosmetics; foundations; concealers; blushes; lipsticks; eyeliners; mascaras; oil removers; color cosmetic removers, powders, and kits thereof.

Hair care compositions include shampoos, rinse-off conditioners, leave-in conditioners and styling aids, gels, sprays, pomades, mousses, waxes, cuticle coats, hair colorants, hair relaxants, hair straighteners, permanents, and kits thereof.

Nail care compositions include color coats, base coats, nail hardeners, and kits thereof.

Health care compositions may be in the form of ointments, creams, gels, mousses, pastes, patches, spray on bandages, foams and/or aerosols or the like, medicament creams, pastes or sprays including anti-acne, dental hygienic, antibiotic, healing promotive, which may be preventative and/or therapeutic medicaments, and kits thereof.

The cosmetic compositions may be used by the standard methods, such as applying them to the human or animal body, e.g. skin or hair, using applicators, brushes, applying by hand, pouring them and/or possibly rubbing or massaging the cosmetic composition onto or into the body. Removal methods, for example for color cosmetics are standard methods understood in the art, including washing, wiping, peeling and the like.

The cosmetic compositions are applied topically to the desired area of the skin or hair in an amount sufficient to provide a satisfactory cleansing or conditioning of the skin or hair. The cosmetic compositions may be diluted with water prior to, during, or after topical application, and then subsequently rinsed or wiped off of the applied surface, for example rinsed off of the applied surface using water or a water-insoluble substrate in combination with water.

This disclosure also comprises a method of treating keratinous substrates, such as hair or skin, by applying to it a cosmetic composition according to certain embodiments of this disclosure.

The cosmetic compositions may be used on hair in a conventional manner. An effective amount of the cosmetic composition for washing or conditioning hair is applied to the hair. Such effective amounts generally range from about 1g to about 50g, optionally from about 1g to about 20g. Application to the hair typically includes working the cosmetic composition through the hair such that most or all of the hair is contacted with the cosmetic composition. These steps can be repeated as many times as desired to achieve the desired benefit.

Benefits obtained from using the cosmetic compositions on hair include one or more of the following benefits: color retention, improvement in coloration process, hair conditioning, softness, detangling ease, silicone deposition, anti-static, anti-frizz, lubricity, shine, strengthening, viscosity, tactile, wet combing, dry combing, straightening, heat protection, styling, or curl retention.

The cosmetic compositions may be used on skin in a conventional manner. An effective amount of the cosmetic composition for the purpose is applied to the skin. Such effective amounts generally range from about 1 mg/cm² to about 3 mg/cm². Application to the skin typically includes working the cosmetic composition into the skin. This method for applying to the skin comprises the steps of contacting the skin with the cosmetic composition in an effective amount and then rubbing the cosmetic composition into the skin. These steps can be repeated as many times as desired to achieve the desired benefit.

Benefits obtained from using the cosmetic compositions on skin include one or more of the following benefits: stability in various formulations (o/w, w/o, anhydrous), utility as an emulsifier, level of hydrophobicity, organic compatibility, substantivity/durability, wash off resistance, interactions with sebum, performance with pigments, pH stability, skin softness, suppleness, moisturization, skin feel, long lasting, long wear, long lasting color uniformity, color enhancement, foam generation, optical effects (soft focus), stabilization of actives.

The cosmetic composition may be used to care for keratinous substrates, to cleanse, to condition, to refresh, to make up, to remove make up, or to fix hair.

### INDUSTRIAL APPLICABILITY

The cross-linked composition of this disclosure is useful for a variety of end applications, and is not limited to any particular one. Examples of suitable applications include use in personal care, household care, and beauty care products. In embodiments having free carboxyl groups, the cross-linked composition can also be used for modifying organic resins or fibers and surface-treating powder. The treated surface shows high affinity with an unctuous agent. Particularly, dispersivity of powder is significantly improved. Therefore, the cross-linked composition can be useful for applications where high dispersivity of a powder is required, e.g., cosmetics such as skincare and makeup products, and coatings. The cross-linked composition can also be used to enhance the aesthetics of personal care formulations for skin care and healthcare by providing a unique sensory profile upon application. The cross-linked composition can provide sensory characteristics such as a velvety, silky, or powdery feel. In addition, the cross-linked composition can be used for providing rheology modification to personal care (e.g. skin, sun, cosmetic, etc.) and healthcare formulations. The cross-linked composition also has excellent formulation versatility. Without being bound or limited to any particular theory, it is thought that potential benefits provided by, or attributable to, the cross-linked composition include, but are not limited to, one or more of the following: film forming, substantivity, durability, pigment/particle suspension and/or modification, long lasting/wear, additional chemistry, actives (e.g. drug) or inactives (e.g. fragrance) delivery/release (e.g. to skin), and combinations thereof. It is thought that carboxyl (or carboxy) groups may also interact with a subject's skin, thus providing long lasting/wear benefits of cosmetic compositions.

It is thought that the carboxy groups in the elastomer structure enhance the skin interaction resulting in significantly improved substantivity, and wash-off resistance especially in the presence of sebum. In various embodiments, the elastomer has a dry, velvety feel unmatched to those of the conventional elastomers. In various embodiments, water uptake of the elastomer is as high as 70% and compatible to organic solvents typically used in personal care applications.

The following examples, illustrating the cross-linked compositions, cosmetic composition, and related methods and components, are intended to illustrate and not to limit the invention.

The following examples are included to demonstrate various embodiments of the invention. It should be appreciated by those of skill in the art that the techniques disclosed in the examples which follow represent techniques discovered by the inventor to function well in the practice of the invention, and thus can be considered to constitute desirable modes for its practice. All percentages are in wt.% and all measurements are conducted at 23ºC unless indicated otherwise. Subscripts and linking groups are as described above unless otherwise defined.

First, a reaction intermediate, which may be referred to as a carbic siloxane intermediate, is formed. Next, the reaction intermediate is used to make a cross-linked composition or elastomer blend.

### Materials to make Carbic Siloxane Intermediate

Organohydrogenpolysiloxane: Si-H polymer supplied by Dow Silicones Corporation of the average formula: MD_{W}D'_{X}M or the average formula:

(CH₃)₃Si-O-[Si(CH₃)₂-O-]_{w}[Si(CH₃)H-O-]ₓSi(CH₃)₃; where w is generally 80 to 100, and x is generally 10 to 20.

The calculated Mw = 7857 and the calculated wt.% SiH = 0.198 wt.%.

Alkenyl functional polycyclic anhydride: carbic anhydride or cis-5-Norbornene-endo-2,3-dicarboxylic anhydride, 97%: 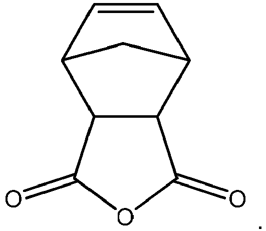
Solvent: isododecane (IDD) or 2,2,4,6,6-pentamethylheptane.
Linear alkene: 1-hexene (CH₂=CHCH₂CH₂CH₂CH₃).
Catalyst: 1 wt.% Platinum solution - prepared from dilution of Pt (IV) compound in IDD.

### Procedure to make Carbic Siloxane Intermediate

### Example 1

To a 500 mL two neck flask is added 3.75 g of carbic anhydride, 12 g of IDD, and 10 µL (∼5 ppm or 174 µg) of catalyst (Pt(IV); 2% solution in xylenes, from Sigma Aldrich). An addition funnel with 20 g of organohydrogenpolysiloxane was put in one port and a septum with a thermocouple was placed in the other. The reaction was heated to 78 °C and then addition of the organohydrogenpolysiloxane was started (right after adding catalyst). Color changed to yellow. After 10 min everything went into solution. Heated for an hour and the temperature slowly reached 110 °C. Sampled for IR and ¹H NMR. No carbic anhydride was left. Reaction flask was cooled to 60 °C and then 6 g of 1-hexene was added. The reaction was not complete after heating for 30 min, but almost done. The reaction was heated for another 30 min and then cooled.

### Example 2

To a 500 mL two neck flask is added 2.5 g of carbic anhydride, 12 g of IDD, and 10 µL (∼5 ppm or 174 µg) of catalyst (Pt(IV); 2% solution in xylenes, from Sigma Aldrich). An addition funnel with 20 g of organohydrogenpolysiloxane was put in one port and a septum with a thermocouple was placed in the other. The reaction was heated to 78 °C and then addition of the organohydrogenpolysiloxane was started (right after adding catalyst). Color changed to yellow. After 10 min everything went into solution. Heated for an hour and the temperature slowly reached 110 °C. Sampled for IR and 1H NMR. No carbic anhydride was left. Reaction flask was cooled to 60 °C and then 6 g of 1-hexene was added. The reaction was not complete after heating for 30 min, but almost done. The reaction was heated for another 30 min and then cooled.

### Example 3

To a 500 mL two neck flask is added 1.25 g of carbic anhydride, 12 g of IDD, and 10 µL (∼5 ppm or 174 µg) of catalyst (Pt(IV); 2% solution in xylenes, from Sigma Aldrich). An addition funnel with 20 g of organohydrogenpolysiloxane was put in one port and a septum with a thermocouple was placed in the other. The reaction was heated to 78 °C and then addition of the organohydrogenpolysiloxane was started (right after adding catalyst). Color changed to yellow. After 10 min everything went into solution. Heated for an hour and the temperature slowly reached 110 °C. Sampled for IR and 1H NMR. No carbic anhydride was left. Reaction flask was cooled to 60 °C and then 6 g of 1-hexene was added. After 30 min another 1HNMR was taken, no SiH was detected.

The reaction intermediates formed in each of Examples 1, 2, and 3 above is of the general formula:

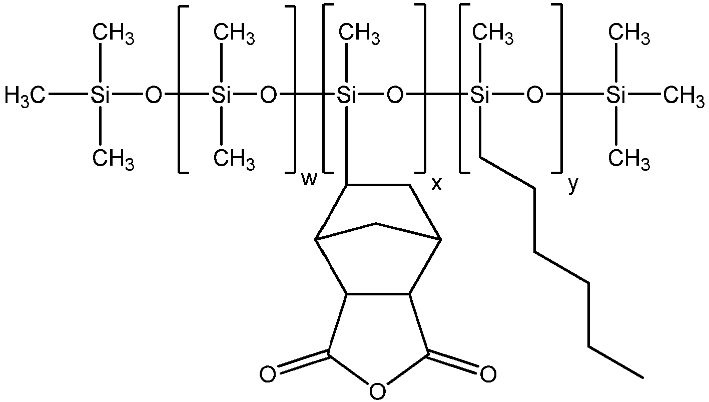

### Materials to make Elastomer Blend

Reaction Intermediate: carbic siloxane intermediates formed in Examples 1, 2, and 3 above.

Polyol: 1,6-hexanediol (HOCH₂CH₂CH₂CH₂CH₂CH₂OH).

Solvent: IDD.

### Procedure to make Elastomer Blend

### Example 4

Place 19.0 g of reaction intermediate, 0.52 g of polyol, and 40.0 g of IDD into a reactor flask with a magnetic stirrer. Heat the reaction to 80 °C and once reaction gels hold at 80 °C for three hours. Cool the reactor flask to room temperature. Remove the gelled material to a laboratory blender and shear the material into a paste.

Further Examples are formed using the procedure of Example 4 above, but with different amounts of components as illustrated in Table 1 below.

**TABLE 1**

| **Component in (g)** | ***Example*** | | |
|---|---|---|---|
| | **5** | **6** | **7** |
| Reaction Intermediate | 31.92 | 32.13 | 39.48 |
| Polyol | 0.88 | 0.8 | 0.52 |
| Solvent | 67.2 | 67.2 | 60 |

The elastomers formed in Examples 4, 5, 6, and 7 above are of the general formula:

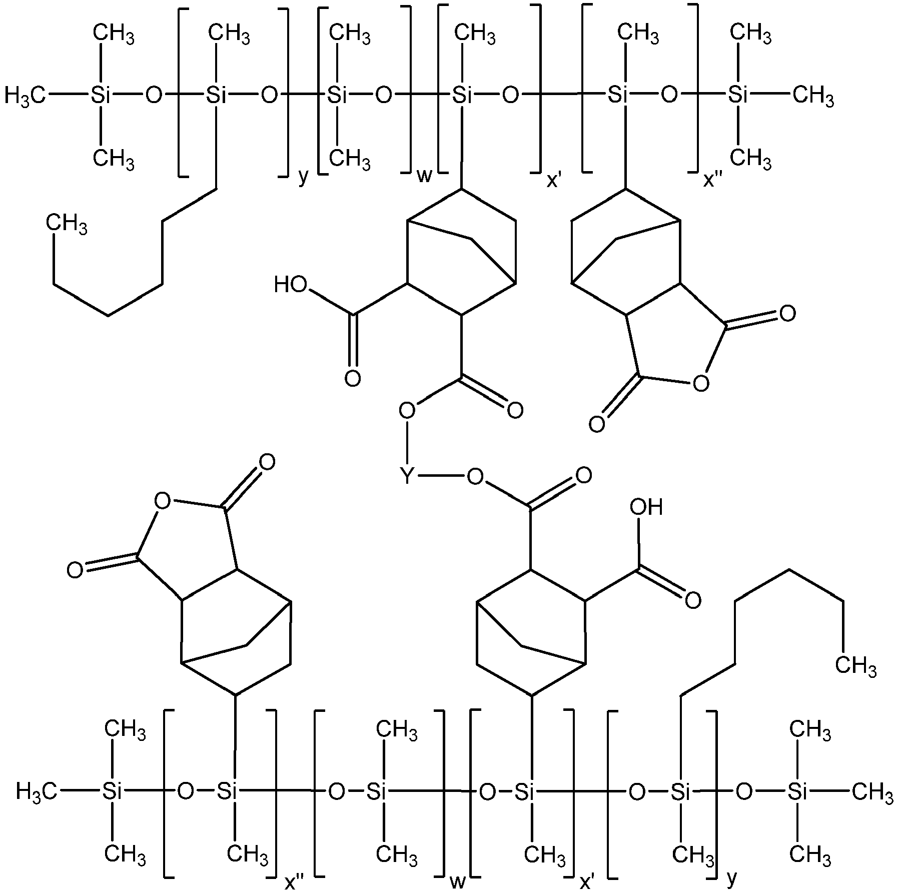

Further examples of elastomers that can be formed follow below.

In this elastomer, the linear alkene is not used and excess pendant polycyclic anhydride groups are present:

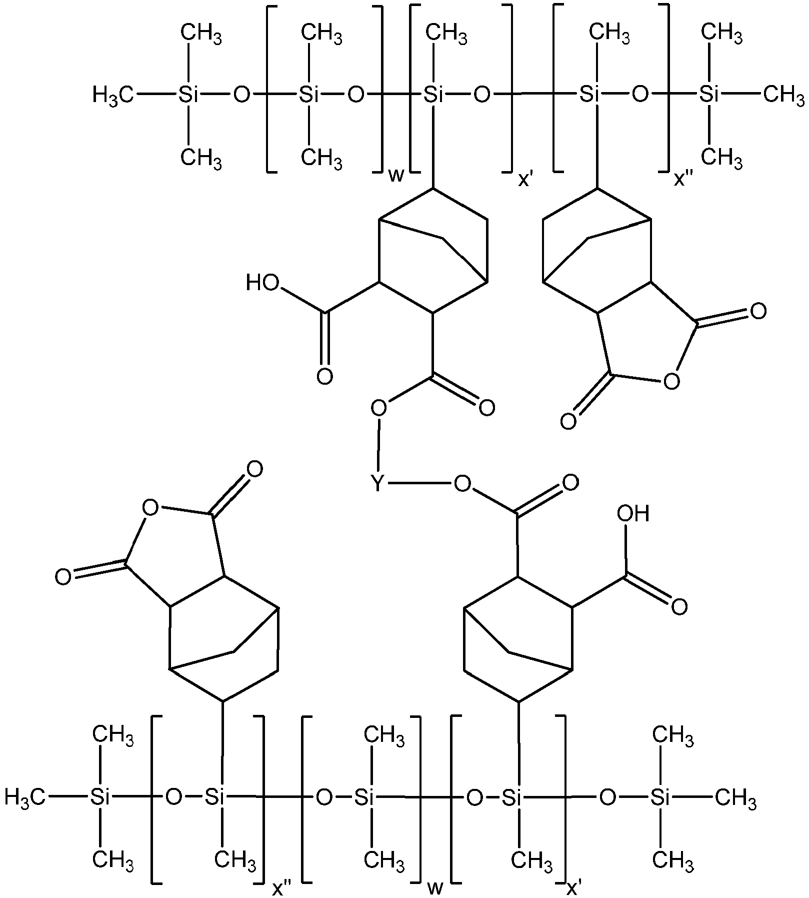

In this elastomer, the linear alkene is not used and one of the siloxanes has a higher number of pendant polycyclic anhydride groups:

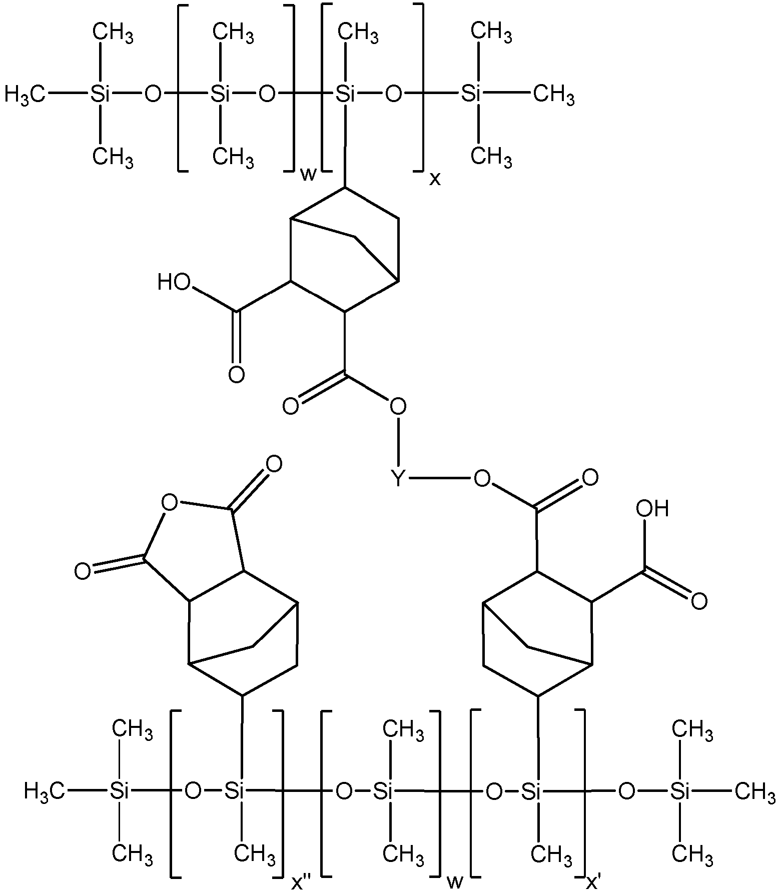

In this elastomer, the linear alkene is not used and all of the pendant polycyclic anhydride groups are cross-linked:

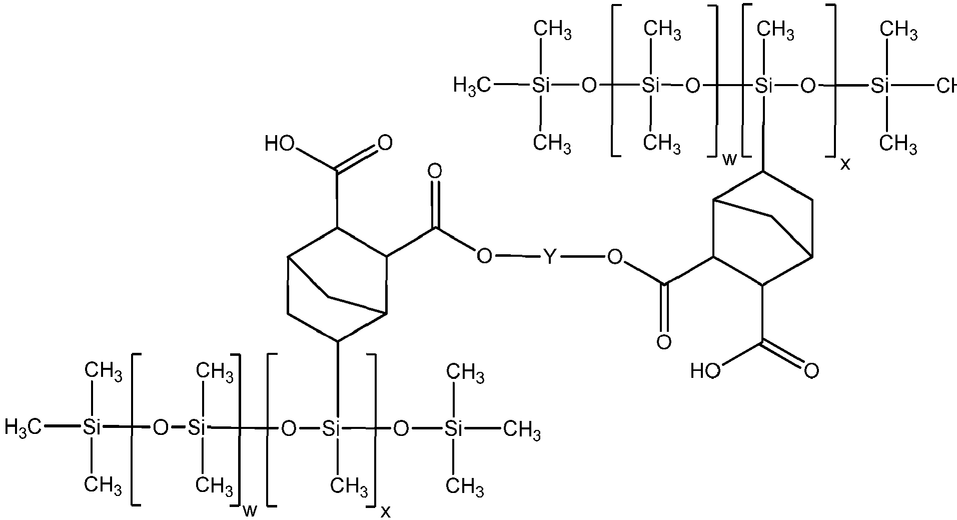

In this elastomer, the linear alkene is used and all of the pendant polycyclic anhydride groups are cross-linked:

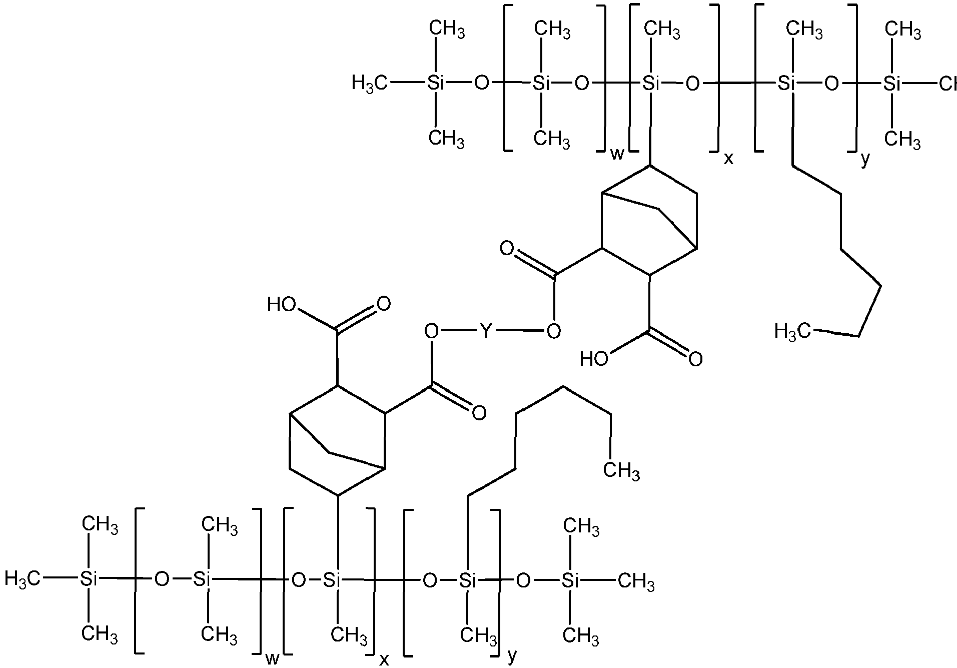

In this elastomer, the linear alkene is not used for one of the siloxanes and all of the pendant polycyclic anhydride groups are cross-linked:

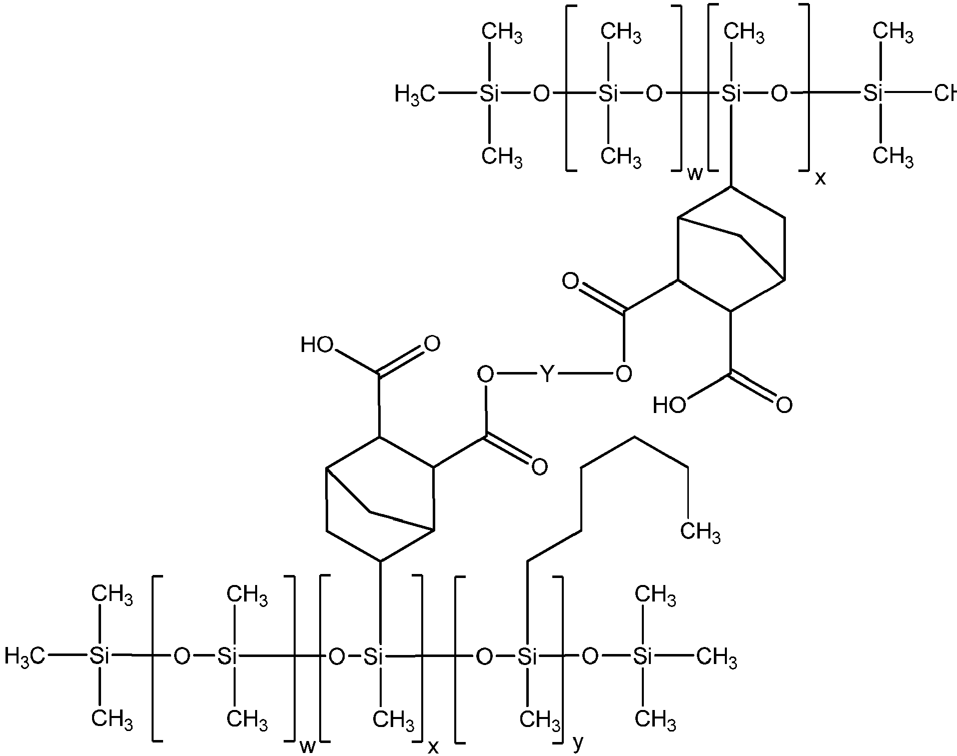

It is to be appreciated that other elastomers are also possible in view of the teachings herein, but are not specifically elucidated herein.

### Odor Detection for 3-hexenoic acid in Elastomer Blend

Samples of the elastomer blends were analyzed by headspace GC/MS in order to determine if by chance 3-hexenoic acid was contained in the sample. 3-hexenoic acid is believed to be a malodorous compound present in conventional elastomer blends, such as those that may be present in the elastomers of U.S. Pat. No. 9,822,221. The analysis did not detect 3-hexenoic acid in any of the samples. The estimated detection limit is less than 1 ppm. No other compounds were targeted.

### Procedure

Each sample was analyzed as received by headspace GC/MS using MS13. Exactly 1.0 g of material was placed in an empty 20 mL headspace vial and crimped closed using a Teflon backed silicone septa. The instrument's mass calibrations were verified to be accurate on the same day the analysis was performed.

### EI GC/MS Instrument Conditions

### Direct Injection Analysis:

Agilent 7890A GC
- GC column: HP-INNOWAX, 15 m × 0.25 mm × 0.25 µm film
- Oven program: 40(3)-200°C(3)@15°/min-260°C(3)@25°C/min; 260°C inlet
   ∘ Post Run: n/a
- Constant helium flow @ 1 mL/min
- 10:1 Split Injection at 240°C Inlet

Headspace Conditions: 5 minutes at 110°C; 110°C Syringe; 1.0 mL Injection

Agilent 5975C MSD
- MS Mode: SIM; m/z = 55, 68, & 114 Da
- Scan Rate: 3.1 Hz
- EI (electron impact) ionization; 230°C Source

Additional Examples are provided below.

### Formation of BTHPA

In a 2L round bottomed flask was placed maleic anhydride (92.0 g, 0.94 mmol) and 910 mL toluene. Then, 1,3,7-octatriene (205 g, 1.88 mmol, 1:1 *cis:trans*) was added to the mixture and was allowed to stir at room temperature for 72h. Afterwards, the solvent and remaining octatriene was removed via rotary evaporation under reduced pressure at 40 °C. The remaining liquid was filtered through a frit and heated at a hot plate temperature of 135 °C under vacuum (50 mTorr) to remove remaining solvent and sublime residual maleic anhydride. Then, the hot plate was set to 180 °C and the desired product was distilled over as a clear colorless liquid which may spontaneously solidify upon standing into a white solid. Yield = 165.5 g (86%); *endo:exo* = 98:2.

### Synthesis of Functionalized Polyorganosiloxane

Synthesis of anhydride functionalized polyorganosiloxane (Polymer A) and carboxy functionalized polyorganosiloxane (Polymer B) using Anhydride **"1"** (e.g. BTHPA) is illustrated in the simplified reaction scheme below:

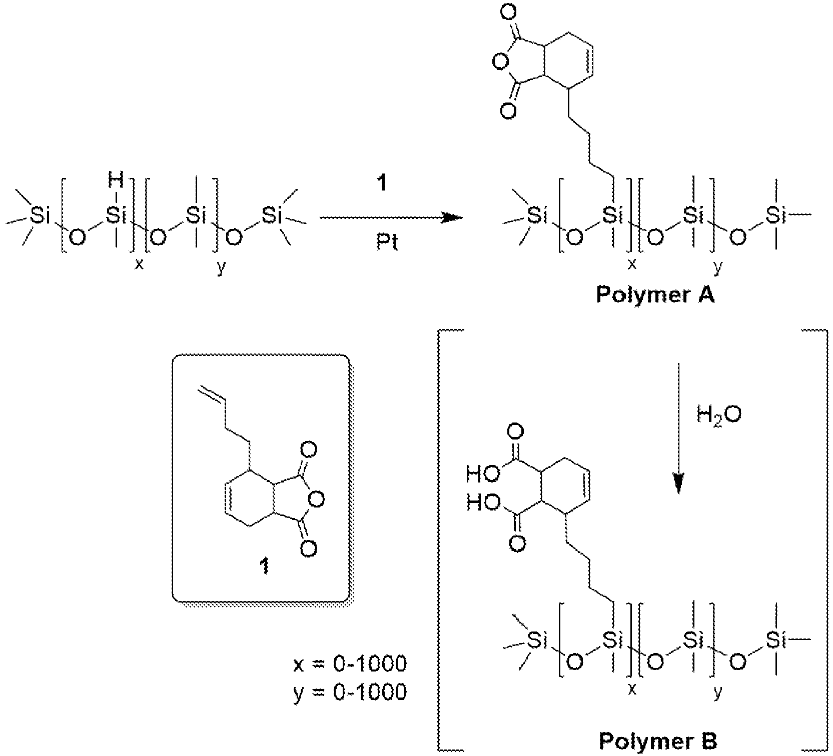

### Synthesis of Polymer A

A flask equipped with a thermometer, stirrer and addition funnel was charged with 20 g of organohydrogenpolysiloxane, 12 g of isododecane (IDD) and 10 µL Karstedt's catalyst (2% Pt in xylenes). The reaction was heated to 50 °C and then 8.5 g of Anhydride **1** was added through an addition funnel and the reaction temperature was maintained at 75 °C for 1 h and then cooled back to room temperature. The reaction was then stirred for another 2 h. ¹H NMR spectroscopy indicates ∼4% SiH left. Quantitative yield.

### Synthesis of Carboxy Fluid

A carboxy fluid similar to Polymer A is formed. To a 250 mL two neck flask is added 20 g of organohydrogenpolysiloxane and 12 g of IDD and 10 µL of catalyst (2% in xylenes). An addition funnel with 8.5 g of Anhydride **1** was put in one port and a septum with a thermocouple was placed in the other. The reaction was heated to 50 °C and then the addition was started lasting 10 minutes. The reaction was heated to 75 °C for 3 h.

### Synthesis of Polymer B

### Method 1

To a closed 20 mL vial was added 2 g of Polymer A, 1 g of tetrahydrofuran and 120 mg of distilled water. The mixture was stirred and heated at 80 °C for 8 h and then the tetrahydrofuran and water were stripped off. IR spectroscopy indicates the anhydride rings were completely opened.

### Method 2

To a closed 20 mL vial was added 2 g of Polymer A, 120 mg of distilled water, and 20 mg of para-toluenesulfonic acid. The mixture was stirred and heated at 80 °C for 2 h and then the water was stripped off. IR spectroscopy indicates the anhydride rings were completely opened.

### Synthesis of Another Functionalized Polyorganosiloxane

Synthesis of another anhydride functionalized polyorganosiloxane (Polymer 1) and carboxy functionalized cross-linked elastomer (Polymer B) using Anhydride **"1"** (e.g. BTHPA), 1-hexene, and 1,6-hexanediol is illustrated in the simplified reaction scheme below:

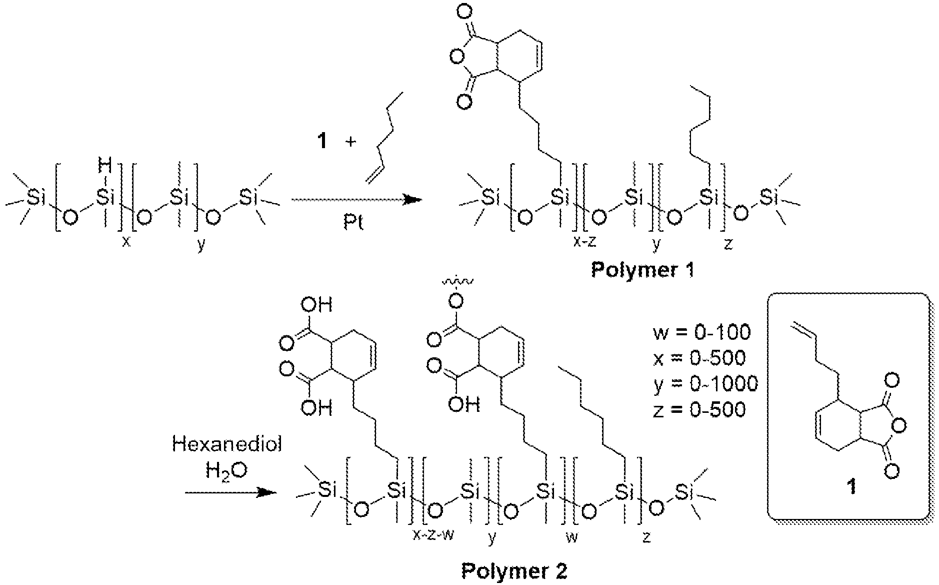

### Synthesis of Polymer 1

A flask equipped with a thermometer, stirrer and addition funnel was charged with 80 g of organohydrogenpolysiloxane, 48 g of IDD and 160 µL Karstedt's catalyst (2% Pt in xylenes). The reaction was heated to 40 °C and then 8.2 mL of 1-hexene was slowly added. The reaction exothermed to 65 °C. 18 g of Anhydride 1 was then added through an addition funnel and the reaction temperature was maintained at 75 °C for 1 h. The reaction was cooled to 60°C and 20 µL Karstedt's catalyst and 1 mL of 1-hexene were added while the reaction temperature was maintained for 30 min. The volatiles were then stripped off at 120 °C under vacuum. ¹H NMR and IR spectroscopy indicate no SiH is left in the polymer. 99 g of product was collected (95% yield).

### Synthesis of Siloxane Intermediate

A siloxane intermediate similar to Polymer 1 is formed. To a 250 mL two neck flask is added 20 g of organohydrogenpolysiloxane, 12 g of IDD and 40 µL catalyst (2% in xylenes). The reaction was heated to 40 °C and then 2.5 mL of 1-hexene was slowly added. The reaction exothermed to 65 °C. An addition funnel with 4.5 g of Anhydride **1** was put in one port and a septum with a thermocouple was placed in the other. After the 1-hexene was added, the addition of the Anhydride **1** was started over 5 minutes. After the addition, the reaction was heated to 75 °C. After one hour the reaction was cooled to 55 °C and added more 1mL 1-hexene and 10 µL Pt was added.

### Synthesis of Polymer 2

Load 8.78 g of Polymer A, 0.22 g of 1,6-hexanediol, and 21.0 g of IDD to a 4 oz. squat glass jar that contains a one inch star shaped magnetic stirrer. Place in a water bath that is at 80 °C and is above a temperature controller/magnetic stirrer. Set the stirring to setting 4 and allow to react until gelled, approximately 2 hours. Once gelled place 4 oz. jar in oven set at 80 °C and allow to cure for an additional three hours. Remove sample from oven and allow to cool to room temperature. The sample is then sheared using a waring blender and additional IDD can be added to achieve the right viscosity or texture.

### Use of Elastomer

The elastomer formed in an Example above, hereinafter referred to as "COOH-elastomer," can be introduced into various cosmetic formulations, with formulations and procedures for forming the particular cosmetic compositions described in U.S. Patent Appl. No. 62/664,654.

The terms "comprising" or "comprise" are used herein in their broadest sense to mean and encompass the notions of "including," "include," "consist(ing) essentially of," and "consist(ing) of." The use of "for example," "e.g.," "such as," and "including" to list illustrative examples does not limit to only the listed examples. Thus, "for example" or "such as" means "for example, but not limited to" or "such as, but not limited to" and encompasses other similar or equivalent examples. The term "about" as used herein serves to reasonably encompass or describe minor variations in numerical values measured by instrumental analysis or as a result of sample handling. Such minor variations may be in the order of ±0-25, ±0-10, ±0-5, or ±0-2.5, % of the numerical values. Further, The term "about" applies to both numerical values when associated with a range of values. Moreover, the term "about" may apply to numerical values even when not explicitly stated.

Generally, as used herein a hyphen "-" or dash "-" in a range of values is "to" or "through"; a ">" is "above" or "greater-than"; a "≥" is "at least" or "greater-than or equal to"; a "<" is "below" or "less-than"; and a "≤" is "at most" or "less-than or equal to."

## Claims

1. A cross-linked composition comprising the reaction product of:
a first siloxane having at least one pendant polycyclic anhydride group;
a second siloxane having at least one pendant polycyclic anhydride group; and
a polyol having at least two hydroxyl groups reactive with the pendant polycyclic anhydride groups of said first and second siloxanes.

2. The cross-linked composition according to claim 1, wherein each of said first and second siloxanes has at least two pendant polycyclic anhydride groups, optionally each of said first and second siloxanes is free of terminal anhydride groups.

3. The cross-linked composition according to claim 1 or 2, wherein each of said first and second siloxanes are polyorganosiloxanes comprising [SiR¹R³-O] units, R¹ is an independently selected substituted or unsubstituted hydrocarbyl group, and R³ comprises, optionally is a polycyclic anhydride group of the following general formula (A1):

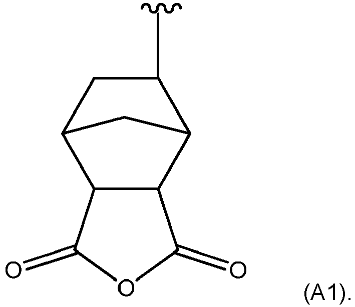

4. The cross-linked composition according to claim 1 or 2, wherein each of said first and second siloxanes are polyorganosiloxanes comprising [SiR¹R³-O] units, R¹ is an independently selected substituted or unsubstituted hydrocarbyl group, and R³ comprises, optionally is a polycyclic anhydride group of the following general formula (A2): 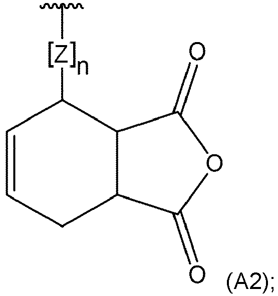 where Z is a divalent linking group, and n is 0 or an integer greater than zero.

5. The cross-linked composition according to any one of the preceding claims, wherein each of said first and second siloxanes is individually an organopolysiloxane of the following general formula (B1):
R⁰R¹₂Si-O-[SiR¹R²-O-]_{w}[SiR¹R³-O-]ₓ[SiR¹R⁴-O-]_{y}SiR¹₂R⁰ (B1);
wherein each R⁰ is an independently selected substituted or unsubstituted hydrocarbyl group or R³; each R¹ is an independently selected substituted or unsubstituted hydrocarbyl group, optionally is an independently selected alkyl group; each R² is an independently selected substituted or unsubstituted hydrocarbyl group, optionally is R¹; R³ is as defined above; R⁴ is a substituted or unsubstituted hydrocarbyl group, optionally is an alkyl group, an aryl group, or a polyether group; w is an integer of from 0 to 1,000, optionally 1 to 300; x is an integer of from 1 to 100, optionally 2 to 75; and y is an integer of from 0 to 1,000, optionally 1 to 300.

6. A cross-linked composition comprising the following general formula (I): 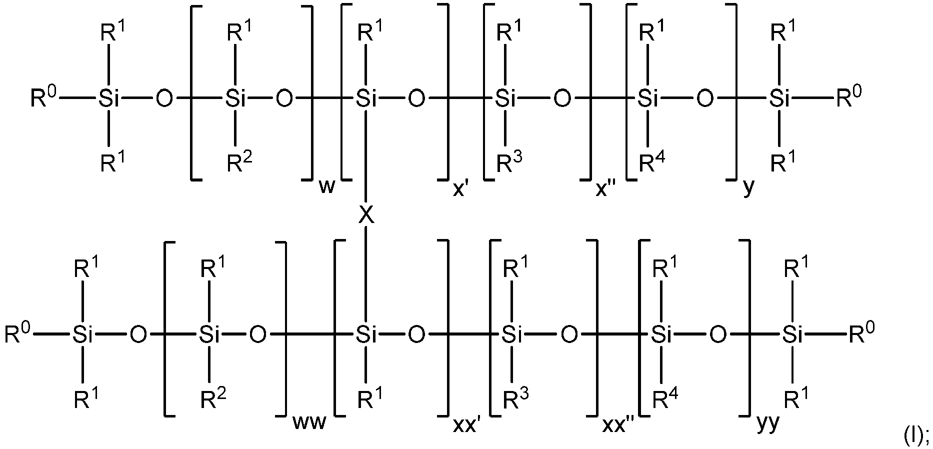 wherein each of R⁰, R¹, and R² is an independently selected substituted or unsubstituted hydrocarbyl group, optionally is an independently selected alkyl group; each R³ comprises, optionally is a polycyclic anhydride group of the following general formula (A1): 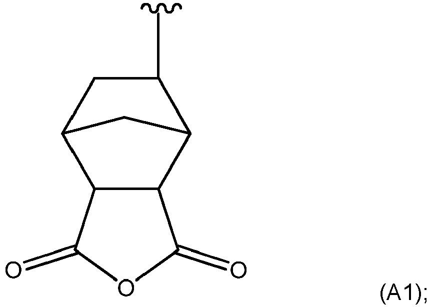 each R⁴ is an independently selected substituted or unsubstituted hydrocarbyl group, optionally is an alkyl group, an aryl group, or a polyether group; each of w and ww is an independently selected integer of from 0 to 1,000, optionally 1 to 300; each of x' and xx' is an independently selected integer ≥1, optionally ≥2; each of x" and xx" is an independently selected integer ≥0, optionally ≥1; the sum of x'+x" is an integer of from 1 to 100, optionally 2 to 75; the sum of xx'+xx" is an integer of from 1 to 100, optionally 1 to 75; each of y and yy is an independently selected integer of from 0 to 1,000, optionally 1 to 300; X comprises, optionally is of the following general formulae (i-a), (i-b) or (i-c); 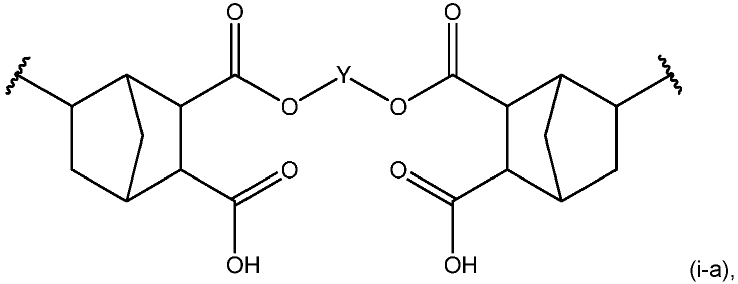 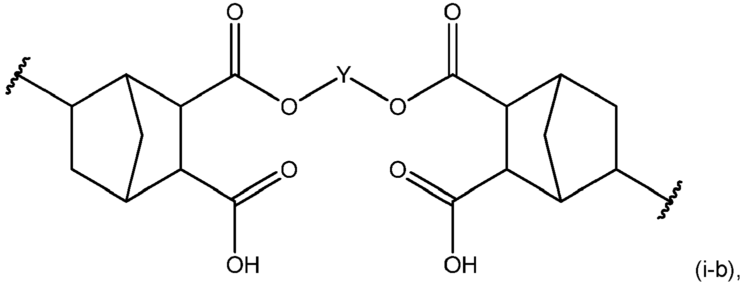 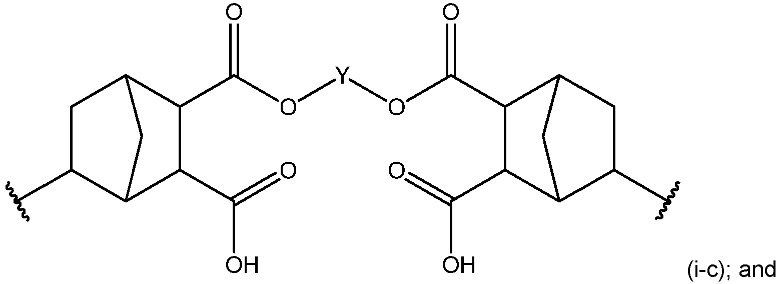 Y is a divalent group formed from a polyol having at least two hydroxyl groyps.

7. A cross-linked composition comprising the following general formula (I): 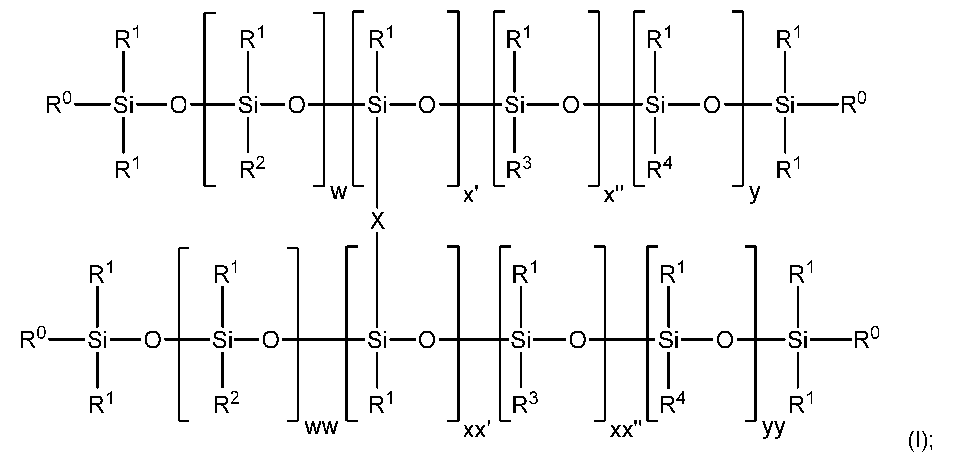 wherein each of R⁰, R¹, and R² is an independently selected substituted or unsubstituted hydrocarbyl group, optionally is an independently selected alkyl group; each R³ comprises, optionally is a polycyclic anhydride group of the following general formula (A2): 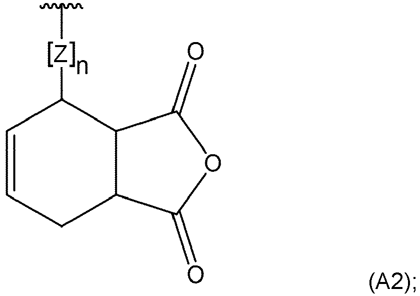 where Z is a divalent linking group, n is 0 or an integer greater than zero; each R⁴ is an independently selected substituted or unsubstituted hydrocarbyl group, optionally is an alkyl group, an aryl group, or a polyether group; each of w and ww is an independently selected integer of from 0 to 1,000, optionally 1 to 300; each of x' and xx' is an independently selected integer ≥1, optionally ≥2; each of x" and xx" is an independently selected integer ≥0, optionally ≥1; the sum of x'+x" is an integer of from 1 to 100, optionally 2 to 75; the sum of xx'+xx" is an integer of from 1 to 100, optionally 1 to 75; each of y and yy is an independently selected integer of from 0 to 1,000, optionally 1 to 300; X comprises, optionally is of the following general formulae (ii-a), (ii-b) or (ii-c); 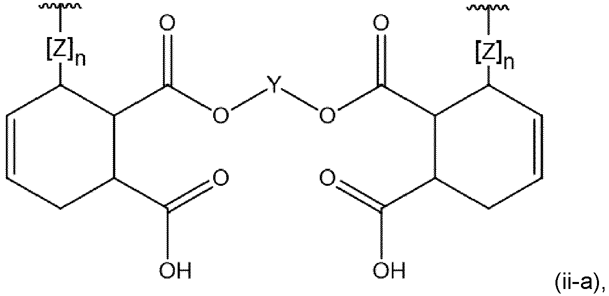 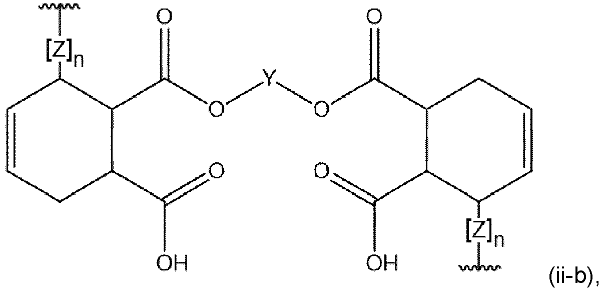 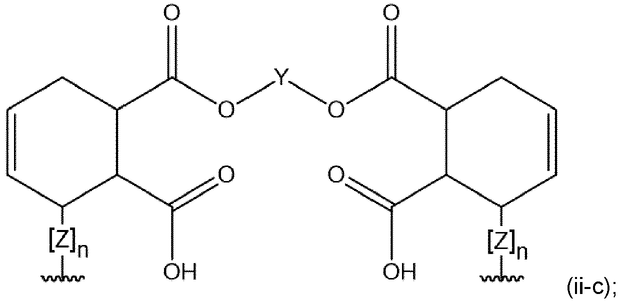 where Z is a divalent linking group, n is 0 or an integer greater than zero, and Y is a divalent group formed from a polyol having at least two hydroxyl groups.

8. The cross-linked composition according to claim 6 or 7, wherein each of R⁰, R¹, and R² is a methyl group; each R⁴ is independently R¹ or an alkyl group having from 2 to 20 carbon atoms; each of w and ww is an independently selected integer of from 50 to 200; the sum of x'+x" is an integer of from 2 to 50; the sum of xx'+xx" is an integer of from 1 to 50; and each of y and yy is an independently selected integer of from 1 to 200.

9. The cross-linked composition according to one of claims 6-8, wherein:
i) each R⁴ is independently an alkyl group having from 4 to 10 carbon atoms, optionally 6 to 8 carbon atoms; and/or
ii) each of w and ww is an independently selected integer of from 75 to 125; each of x' and xx' is an independently selected integer of from 2 to 20; each of x" and xx" is an independently selected integer of from 1 to 20; and each of y and yy is an independently selected integer of from 1 to 20.

10. The cross-linked composition according to any one of the preceding claims, wherein said polyol has the following general formula (II):
HO-R⁵-OH (II)
; wherein R⁵ comprises at least one of a hydrocarbylene, a heterohydrocarbylene, or an organoheterylene group, optionally is a hydrocarbylene group having from 1 to 20 carbon atoms.

11. The cross-linked composition according to any one of the preceding claims, wherein said polyol comprises a polysiloxane having at least two hydroxyl groups, optionally said polysiloxane is of the following general formula (III):
HO-[Z]_{d}-[SiR⁶R⁷-O-]ₐ[SiR⁶R⁸-O-]_{b}[SiR⁶R⁷-O-]_{c}Si-[Z]_{d}-OH (III)
; wherein each of R⁶ and R⁷ is an independently selected substituted or unsubstituted hydrocarbyl group; R⁸ comprises an independently selected substituted or unsubstituted hydrocarbyl group or -[Z]_{d}-OH; each Z independently comprises at least one of a hydrocarbylene, heterohydrocarbylene, or organoheterylene group, optionally each Z is a hydrocarbylene group having from 1 to 20 carbon atoms; a is an integer selected from 0 to 1,000; b is an integer selected from 1 to 200; c is an integer selected from 0 to 1,000; and each d is independently 0 or 1, optionally is 1.

12. A cross-linked composition comprising the reaction product of:
a first siloxane having at least one pendant anhydride group;
a second siloxane having at least one pendant anhydride group; and
a polyol having at least two hydroxyl groups reactive with the pendant anhydride groups of said first and second siloxanes;
wherein at least one of the pendant anhydride groups comprises an unsaturated bond.

13. A personal care composition comprising:
(a) a cross-linked composition having at least two carboxyl groups; and
(b) a least one cosmetic component;
(c) optionally in a cosmetically acceptable medium;
wherein said cross-linked composition is as set forth in any one of the preceding claims.

14. A method of forming a cross-linked composition, said method comprising:
providing an organohydrogenpolysiloxane of the following general formula (B):
R⁰R¹₂Si-O-[SiR¹R²-O-]_{w}[SiHR¹-O-]ₓ[SiR¹R⁹-O-]_{y}SiR¹₂R⁰ (B)
;
wherein each R⁰ is an independently selected substituted or unsubstituted hydrocarbyl group or a hydrogen, optionally is an independently selected alkyl group; each of R¹ and R² is an independently selected substituted or unsubstituted hydrocarbyl group, optionally is an independently selected alkyl group; R⁹ is a substituted or unsubstituted hydrocarbyl group or a hydrogen, optionally is an alkyl group, an aryl group, or a polyether group; w is an integer of from 0 to 1,000, optionally 1 to 300; x is an integer of from 1 to 100, optionally 2 to 75; and y is an integer of from 0 to 1,000, optionally 1 to 300;
providing an alkenyl functional polycyclic anhydride;
optionally, providing a linear alkene having 2 to 20 carbon atoms;
providing a polyol having at least two hydroxyl groups;
combining the organohydrogenpolysiloxane and the alkenyl functional polycyclic anhydride, and optionally the linear alkene, in the presence of a hydrosilylation catalyst to form a reaction intermediate comprising siloxanes having at least one pendant polycyclic anhydride group; and
combining the siloxanes and the polyol to form the cross-linked composition.

15. A polyorganosiloxane composition comprising the reaction product of:
a polycyclic anhydride compound of the following general formula: 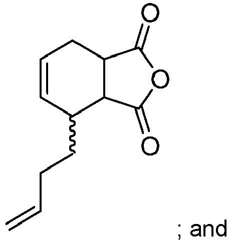 an organosilicon compound having at least one silicon-bonded hydrogen atom.

## Patentansprüche

1. Eine vernetzte Zusammensetzung, die das Reaktionsprodukt aus Folgendem beinhaltet:
einem ersten Siloxan, das mindestens eine seitenständige polycyclische Anhydridgruppe aufweist;
einem zweiten Siloxan, das mindestens eine seitenständige polycyclische Anhydridgruppe aufweist; und
einem Polyol, das mindestens zwei Hydroxylgruppen aufweist, die mit den seitenständigen polycyclischen Anhydridgruppen des ersten und des zweiten Siloxans reaktiv sind.

2. Vernetzte Zusammensetzung gemäß Anspruch 1, wobei jedes von dem ersten und dem zweiten Siloxan mindestens zwei seitenständige polycyclische Anhydridgruppen aufweist, wobei jedes von dem ersten und dem zweiten Siloxan optional frei von endständigen Anhydridgruppen ist.

3. Vernetzte Zusammensetzung gemäß Anspruch 1 oder 2, wobei jedes von dem ersten und dem zweiten Siloxan ein Polyorganosiloxan ist, das [SiR¹R³-O]-Einheiten beinhaltet, R¹ eine unabhängig ausgewählte substituierte oder unsubstituierte Hydrocarbylgruppe ist und R³ eine polycyclische Anhydridgruppe der folgenden allgemeinen Formel (A1) beinhaltet, optional ist:

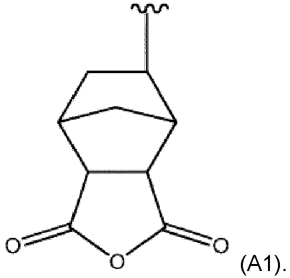

4. Vernetzte Zusammensetzung gemäß Anspruch 1 oder 2, wobei jedes von dem ersten und dem zweiten Siloxan ein Polyorganosiloxan ist, das [SiR¹R³-O]-Einheiten beinhaltet, R¹ eine unabhängig ausgewählte substituierte oder unsubstituierte Hydrocarbylgruppe ist und R³ eine polycyclische Anhydridgruppe der folgenden allgemeinen Formel (A2) beinhaltet, optional ist: 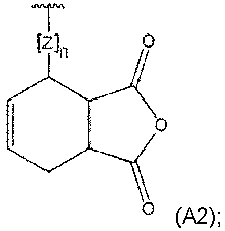 wobei Z eine zweiwertige Verknüpfungsgruppe ist und n 0 oder eine ganze Zahl von größer als null ist.

5. Vernetzte Zusammensetzung gemäß einem der vorhergehenden Ansprüche, wobei jedes von dem ersten und dem zweiten Siloxan jeweils einzeln ein Organopolysiloxan der folgenden allgemeinen Formel (B1) ist:
R⁰R¹₂Si-O-[SiR¹R²-O-]_{w}[SiR¹R³-O-]ₓ[SiR¹R⁴-O-]_{y}SiR¹₂R⁰ (B1)
; wobei jedes R⁰ eine unabhängig ausgewählte substituierte oder unsubstituierte Hydrocarbylgruppe oder R³ ist; jedes R¹ eine unabhängig ausgewählte substituierte oder unsubstituierte Hydrocarbylgruppe ist, optional eine unabhängig ausgewählte Alkylgruppe ist; jedes R² eine unabhängig ausgewählte substituierte oder
unsubstituierte Hydrocarbylgruppe ist, optional R¹ ist; R³ wie oben definiert ist; R⁴ eine substituierte oder unsubstituierte Hydrocarbylgruppe ist, optional eine Alkylgruppe, eine Arylgruppe oder eine Polyethergruppe ist; w eine ganze Zahl von 0 bis 1 000, optional 1 bis 300 ist; x eine ganze Zahl von 1 bis 100, optional 2 bis 75 ist; und y eine ganze Zahl von 0 bis 1 000, optional 1 bis 300 ist.

6. Eine vernetzte Zusammensetzung, die die folgende allgemeine Formel (I) beinhaltet: 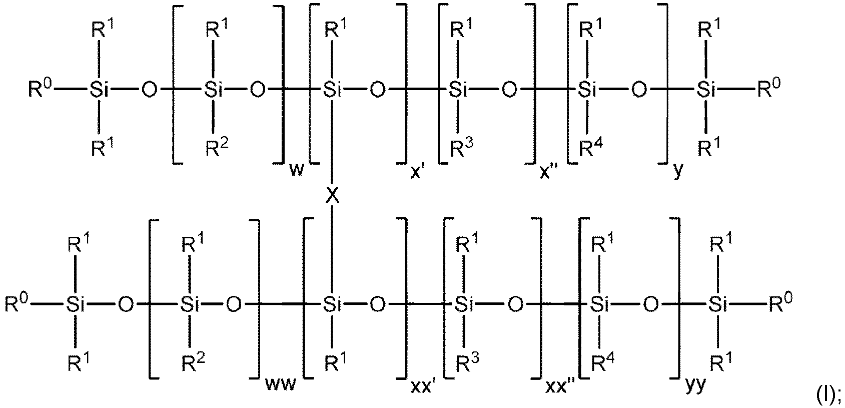 wobei jedes von R⁰, R¹ und R² eine unabhängig ausgewählte substituierte oder unsubstituierte Hydrocarbylgruppe ist, optional eine unabhängig ausgewählte Alkylgruppe ist; jedes R³ eine polycyclische Anhydridgruppe der folgenden allgemeinen Formel (A1) beinhaltet, optional ist: 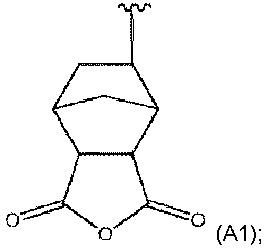 jedes R⁴ eine unabhängig ausgewählte substituierte oder unsubstituierte Hydrocarbylgruppe ist, optional eine Alkylgruppe, eine Arylgruppe oder eine Polyethergruppe ist; jedes von w und ww eine unabhängig ausgewählte ganze Zahl von 0 bis 1 000, optional 1 bis 300 ist; jedes von x' und xx' eine unabhängig ausgewählte ganze Zahl ≥ 1, optional ≥ 2 ist; jedes von x" und xx" eine unabhängig ausgewählte ganze Zahl ≥ 0, optional ≥ 1 ist; die Summe von x' + x" eine ganze Zahl von 1 bis 100, optional 2 bis 75 ist; die Summe von xx' + xx" eine ganze Zahl von 1 bis 100, optional 1 bis 75 ist; jedes von y und yy eine unabhängig ausgewählte ganze Zahl von 0 bis 1 000, optional 1 bis 300 ist;
X die folgenden allgemeinen Formeln (i-a), (i-b) oder (i-c) beinhaltet, optional ist: 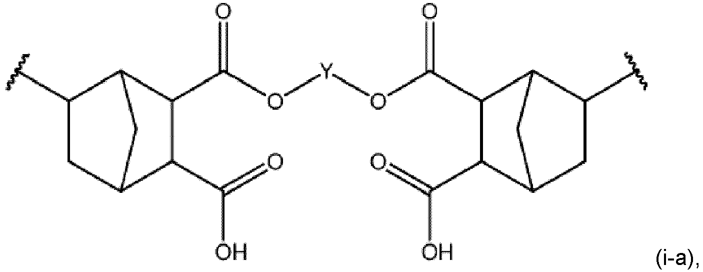 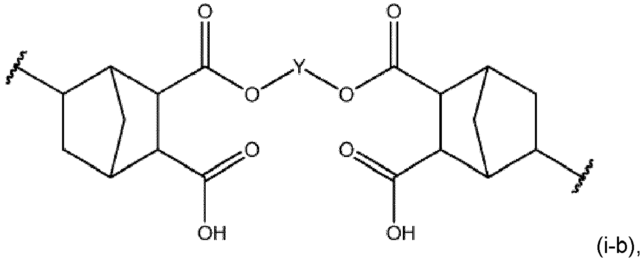 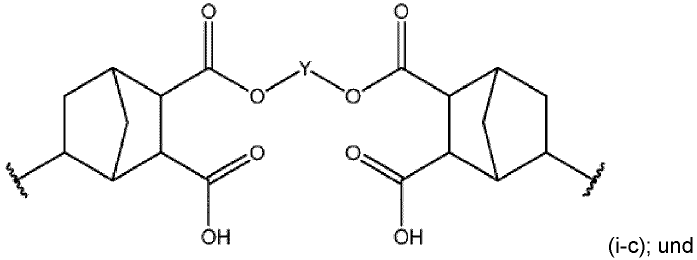 Y eine zweiwertige Gruppe ist, die aus einem Polyol mit mindestens zwei Hydroxylgruppen gebildet ist.

7. Eine vernetzte Zusammensetzung, die die folgende allgemeine Formel (I) beinhaltet: 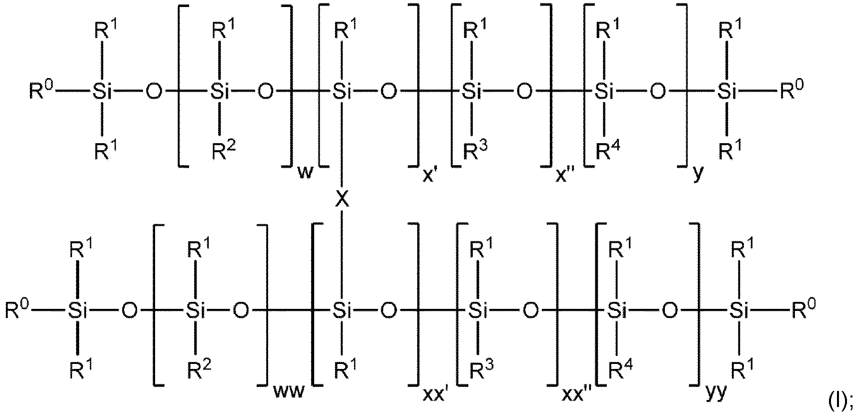 wobei jedes von R⁰, R¹ und R² eine unabhängig ausgewählte substituierte oder unsubstituierte Hydrocarbylgruppe ist, optional eine unabhängig ausgewählte Alkylgruppe ist; jedes R³ eine polycyclische Anhydridgruppe der folgenden allgemeinen Formel (A2) beinhaltet, optional ist: 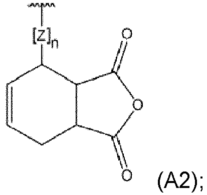 wobei Z eine zweiwertige Verknüpfungsgruppe ist, n 0 oder eine ganze Zahl von größer als null ist; jedes R⁴ eine unabhängig ausgewählte substituierte oder unsubstituierte Hydrocarbylgruppe ist, optional eine Alkylgruppe, eine Arylgruppe oder eine Polyethergruppe ist; jedes von w und ww eine unabhängig ausgewählte ganze Zahl von 0 bis 1 000, optional 1 bis 300 ist; jedes von x' und xx' eine unabhängig ausgewählte ganze Zahl ≥ 1, optional ≥ 2 ist; jedes von x" und xx" eine unabhängig ausgewählte ganze Zahl ≥ 0, optional ≥ 1 ist; die Summe von x' + x" eine ganze Zahl von 1 bis 100, optional 2 bis 75 ist; die Summe von xx' + xx" eine ganze Zahl von 1 bis 100, optional 1 bis 75 ist; jedes von y und yy eine unabhängig ausgewählte ganze Zahl von 0 bis 1 000, optional 1 bis 300 ist; X die folgenden allgemeinen Formeln (ii-a), (ii-b) oder (ii-c) beinhaltet, optional ist: 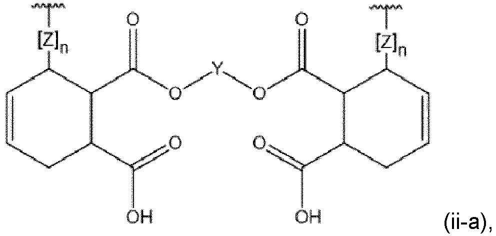 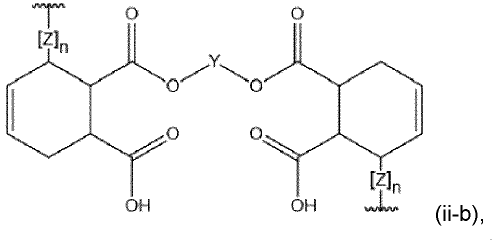 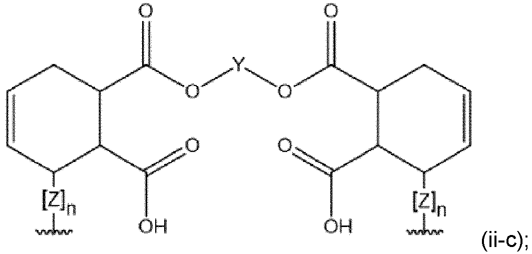 wobei Z eine zweiwertige Verknüpfungsgruppe ist, n 0 oder eine ganze Zahl von größer als null ist und Y eine zweiwertige Gruppe ist, die aus einem Polyol mit mindestens zwei Hydroxylgruppen gebildet ist.

8. Vernetzte Zusammensetzung gemäß Anspruch 6 oder 7, wobei jedes von R⁰, R¹ und R² eine Methylgruppe ist; jedes R⁴ unabhängig R¹ oder eine Alkylgruppe mit 2 bis 20 Kohlenstoffatomen ist; jedes von w und ww eine unabhängig ausgewählte ganze Zahl von 50 bis 200 ist; die Summe von x' + x" eine ganze Zahl von 2 bis 50 ist; die Summe von xx' + xx" eine ganze Zahl von 1 bis 50 ist; und jedes von y und yy eine unabhängig ausgewählte ganze Zahl von 1 bis 200 ist.

9. Vernetzte Zusammensetzung gemäß einem der Ansprüche 6-8, wobei:
i) jedes R⁴ unabhängig eine Alkylgruppe mit 4 bis 10 Kohlenstoffatomen, optional 6 bis 8 Kohlenstoffatomen ist; und/oder
ii) jedes von w und ww eine unabhängig ausgewählte ganze Zahl von 75 bis 125 ist; jedes von x' und xx' eine unabhängig ausgewählte ganze Zahl von 2 bis 20 ist; jedes von x" und xx" eine unabhängig ausgewählte ganze Zahl von 1 bis 20 ist; und jedes von y und yy eine unabhängig ausgewählte ganze Zahl von 1 bis 20 ist.

10. Vernetzte Zusammensetzung gemäß einem der vorhergehenden Ansprüche, wobei das Polyol die folgende allgemeine Formel (II) aufweist:
HO-R⁵-OH (II)
; wobei R⁵ mindestens eine von einer Hydrocarbylen-, einer Heterohydrocarbylen- oder einer Organoheterylengruppe beinhaltet, optional eine Hydrocarbylengruppe mit 1 bis 20 Kohlenstoffatomen ist.

11. Vernetzte Zusammensetzung gemäß einem der vorhergehenden Ansprüche, wobei das Polyol ein Polysiloxan mit mindestens zwei Hydroxylgruppen beinhaltet, wobei das Polysiloxan optional die folgende allgemeine Formel (III) aufweist:
HO-[Z]_{d}-[SiR⁶R⁷-O-]ₐ[SiR⁶R⁸-O-]_{b}[SiR⁶R⁷-O-]_{c}Si-[Z]_{d}-OH (III)
; wobei jedes von R⁶ und R⁷ eine unabhängig ausgewählte substituierte oder unsubstituierte Hydrocarbylgruppe ist; R⁸ eine unabhängig ausgewählte substituierte oder unsubstituierte Hydrocarbylgruppe oder-[Z]_{d}-OH beinhaltet; jedes Z unabhängig mindestens eine von einer Hydrocarbylen-, Heterohydrocarbylen- oder Organoheterylengruppe beinhaltet, jedes Z optional eine Hydrocarbylengruppe mit 1 bis 20 Kohlenstoffatomen ist; a eine ganze Zahl, ausgewählt aus 0 bis 1 000 ist; b eine ganze Zahl, ausgewählt aus 1 bis 200 ist; c eine ganze Zahl, ausgewählt aus 0 bis 1 000 ist; und jedes d unabhängig 0 oder 1 ist, optional 1 ist.

12. Eine vernetzte Zusammensetzung, die das Reaktionsprodukt aus Folgendem beinhaltet:
einem ersten Siloxan, das mindestens eine seitenständige Anhydridgruppe aufweist;
einem zweiten Siloxan, das mindestens eine seitenständige Anhydridgruppe aufweist; und
einem Polyol, das mindestens zwei Hydroxylgruppen aufweist, die mit den seitenständigen Anhydridgruppen des ersten und des zweiten Siloxans reaktiv sind;
wobei mindestens eine der seitenständigen Anhydridgruppen eine ungesättigte Bindung beinhaltet.

13. Eine Körperpflegezusammensetzung, die Folgendes beinhaltet:
(a) eine vernetzte Zusammensetzung mit mindestens zwei Carboxylgruppen; und
(b) mindestens eine kosmetische Komponente;
(c) optional in einem kosmetisch akzeptablen Medium;
wobei die vernetzte Zusammensetzung wie in einem der vorhergehenden Ansprüche angegeben ist.

14. Ein Verfahren zum Bilden einer vernetzten Zusammensetzung, wobei das Verfahren Folgendes beinhaltet:
Bereitstellen eines Organohydrogenpolysiloxans der folgenden allgemeinen Formel (B):
R⁰R¹₂Si-O-[SiR¹R²-O-]_{w}[SiHR¹-O-]ₓ[SiR¹R⁹-O-]_{y}SiR¹₂R⁰ (B)
;
wobei jedes R⁰ eine unabhängig ausgewählte substituierte oder unsubstituierte Hydrocarbylgruppe oder ein Wasserstoff ist, optional eine unabhängig ausgewählte Alkylgruppe ist; jedes von R¹ und R² eine unabhängig ausgewählte substituierte oder
unsubstituierte Hydrocarbylgruppe ist, optional eine unabhängig ausgewählte Alkylgruppe ist; R⁹ eine substituierte oder unsubstituierte Hydrocarbylgruppe oder ein Wasserstoff ist, optional eine Alkylgruppe, eine Arylgruppe oder eine Polyethergruppe ist; w eine ganze Zahl von 0 bis 1 000, optional 1 bis 300 ist; x eine ganze Zahl von 1 bis 100, optional 2 bis 75 ist; und y eine ganze Zahl von 0 bis 1 000, optional 1 bis 300 ist;
Bereitstellen eines alkenylfunktionellen polycyclischen Anhydrids;
optional Bereitstellen eines linearen Alkens mit 2 bis 20 Kohlenstoffatomen;
Bereitstellen eines Polyols mit mindestens zwei Hydroxylgruppen;
Kombinieren des Organohydrogenpolysiloxans und des alkenylfunktionellen polycyclischen Anhydrids und optional des linearen Alkens in Gegenwart eines Hydrosilylierungskatalysators zur Bildung eines Reaktionszwischenprodukts, das Siloxane mit mindestens einer seitenständigen polycyclischen Anhydridgruppe beinhaltet; und Kombinieren der Siloxane und des Polyols, um die vernetzte Zusammensetzung zu bilden.

15. Eine Polyorganosiloxanzusammensetzung, die das Reaktionsprodukt aus Folgendem beinhaltet:
einer polycyclischen Anhydridverbindung der folgenden allgemeinen Formel: 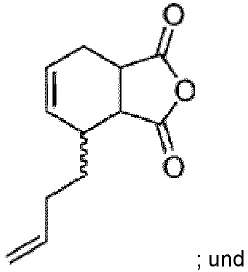 einer Organosiliciumverbindung mit mindestens einem siliciumgebundenen Wasserstoffatom.

## Revendications

1. Une composition réticulée comprenant le produit de réaction :
d'un premier siloxane ayant au moins un groupe anhydride polycyclique pendant ;
d'un deuxième siloxane ayant au moins un groupe anhydride polycyclique pendant ; et
d'un polyol ayant au moins deux groupes hydroxyle qui réagissent avec les groupes anhydride polycycliques pendants desdits premier et deuxième siloxanes.

2. La composition réticulée selon la revendication 1, dans laquelle chacun desdits premier et deuxième siloxanes a au moins deux groupes anhydride polycycliques pendants, facultativement chacun desdits premier et deuxième siloxanes est exempt de groupes anhydride terminaux.

3. La composition réticulée selon la revendication 1 ou la revendication 2, dans laquelle lesdits premier et deuxième siloxanes sont chacun des polyorganosiloxanes comprenant des unités [SiR¹R³-O], R¹ est un groupe hydrocarbyle substitué ou non substitué sélectionné indépendamment, et R³ comprend, facultativement est, un groupe anhydride polycyclique de la formule générale (A1) suivante :

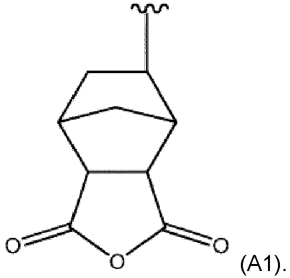

4. La composition réticulée selon la revendication 1 ou la revendication 2, dans laquelle lesdits premier et deuxième siloxanes sont chacun des polyorganosiloxanes comprenant des unités [SiR¹R³-O], R¹ est un groupe hydrocarbyle substitué ou non substitué sélectionné indépendamment, et R³ comprend, facultativement est, un groupe anhydride polycyclique de la formule générale (A2) suivante : 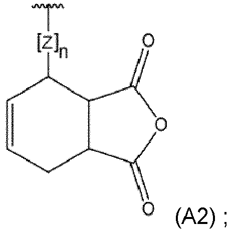 où Z est un groupe de liaison divalent, et n vaut 0 ou un nombre entier supérieur à zéro.

5. La composition réticulée selon n'importe laquelle des revendications précédentes, dans laquelle chacun desdits premier et deuxième siloxanes est individuellement un organopolysiloxane de la formule générale (B1) suivante :
R⁰R¹₂Si-O-[SiR¹R²-O-]_{w}[SiR¹R³-O-]ₓ[SiR¹R⁴-O-]_{y}SiR¹₂R⁰ (B1)
; dans laquelle chaque R⁰ est un groupe hydrocarbyle substitué ou non substitué sélectionné indépendamment ou R³ ; chaque R¹ est un groupe hydrocarbyle substitué ou non substitué sélectionné indépendamment, facultativement est un groupe alkyle sélectionné indépendamment ; chaque R² est un groupe hydrocarbyle substitué ou non substitué sélectionné indépendamment, facultativement est R¹ ; R³ est tel que défini ci-dessus ; R⁴ est un groupe hydrocarbyle substitué ou non substitué, facultativement est un groupe alkyle, un groupe aryle, ou un groupe polyéther ; w est un nombre entier allant de 0 à 1 000, facultativement de 1 à 300 ; x est un nombre entier allant de 1 à 100, facultativement de 2 à 75 ; et y est un nombre entier allant de 0 à 1 000, facultativement de 1 à 300.

6. Une composition réticulée comprenant la formule générale (I) suivante : 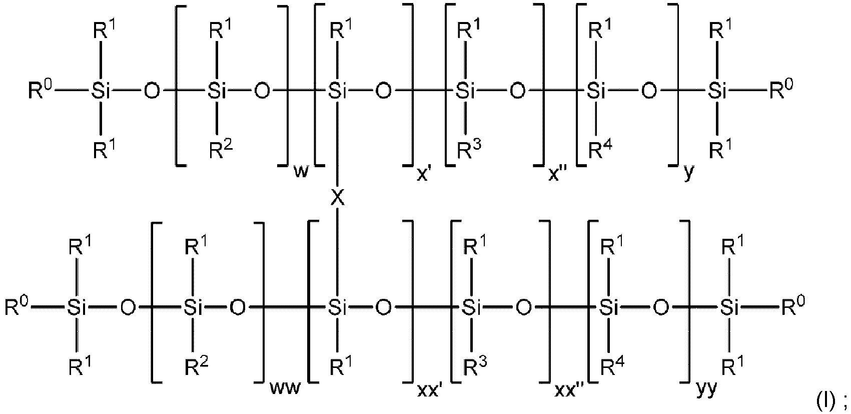 dans laquelle chacun des R⁰, R¹, et R² est un groupe hydrocarbyle substitué ou non substitué sélectionné indépendamment, facultativement est un groupe alkyle sélectionné indépendamment ; chaque R³ comprend, facultativement est, un groupe anhydride polycyclique de la formule générale (A1) suivante : 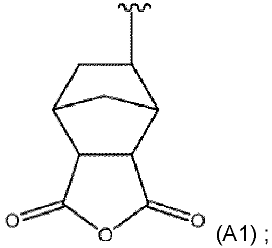 chaque R⁴ est un groupe hydrocarbyle substitué ou non substitué sélectionné indépendamment, facultativement est un groupe alkyle, un groupe aryle, ou un groupe polyéther ; chacun des w et ww est un nombre entier sélectionné indépendamment allant de 0 à 1 000, facultativement de 1 à 300 ; chacun des x' et xx' est un nombre entier sélectionné indépendamment ≥ 1, facultativement ≥ 2 ; chacun des x" et xx" est un nombre entier sélectionné indépendamment ≥ 0, facultativement ≥ 1 ; la somme de x' + x" est un nombre entier allant de 1 à 100, facultativement de 2 à 75 ; la somme de xx' + xx" est un nombre entier allant de 1 à 100, facultativement de 1 à 75 ; chacun des y et yy est un nombre entier sélectionné indépendamment allant de 0 à 1 000, facultativement de 1 à 300 ;
X comprend, facultativement est, des formules générales (i-a), (i-b) ou (i-c) suivantes : 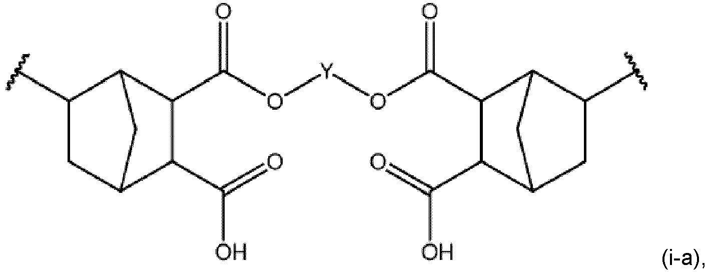 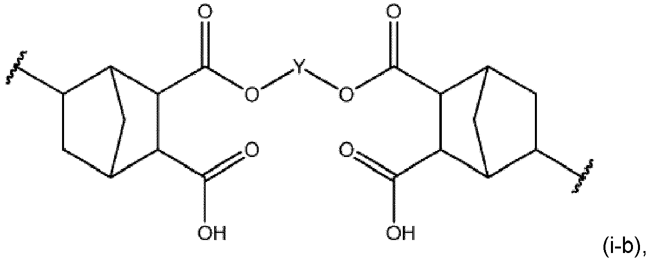 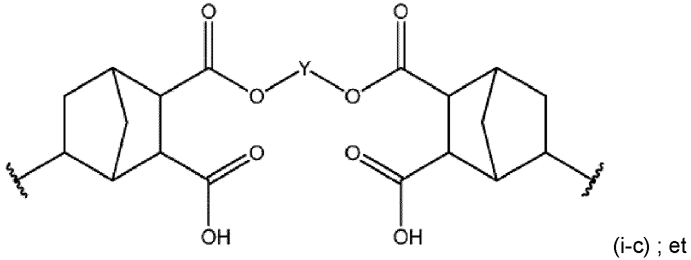 Y est un groupe divalent formé à partir d'un polyol ayant au moins deux groupes hydroxyle.

7. Une composition réticulée comprenant la formule générale (I) suivante : 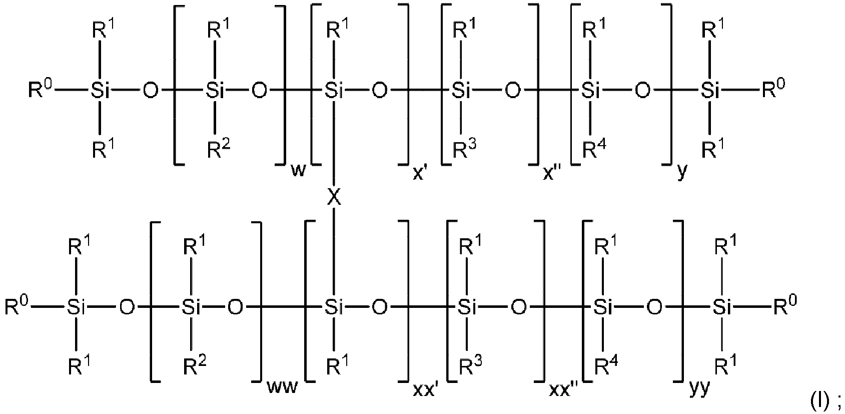 dans laquelle chacun des R⁰, R¹, et R² est un groupe hydrocarbyle substitué ou non substitué sélectionné indépendamment, facultativement est un groupe alkyle sélectionné indépendamment ; chaque R³ comprend, facultativement est, un groupe anhydride polycyclique de la formule générale (A2) suivante : 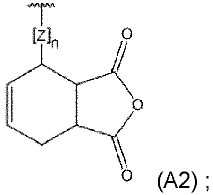 où Z est un groupe de liaison divalent, n vaut 0 ou un nombre entier supérieur à zéro ; chaque R⁴ est un groupe hydrocarbyle substitué ou non substitué sélectionné indépendamment, facultativement est un groupe alkyle, un groupe aryle, ou un groupe polyéther ; chacun des w et ww est un nombre entier sélectionné indépendamment allant de 0 à 1 000, facultativement de 1 à 300 ; chacun des x' et xx' est un nombre entier sélectionné indépendamment ≥ 1, facultativement ≥ 2 ; chacun des x" et xx" est un nombre entier sélectionné indépendamment ≥ 0, facultativement ≥ 1 ; la somme de x' + x" est un nombre entier allant de 1 à 100, facultativement de 2 à 75 ; la somme de xx' + xx" est un nombre entier allant de 1 à 100, facultativement de 1 à 75 ; chacun des y et yy est un nombre entier sélectionné indépendamment allant de 0 à 1 000, facultativement de 1 à 300 ;
X comprend, facultativement est, des formules générales (ii-a), (ii-b) ou (ii-c) suivantes : 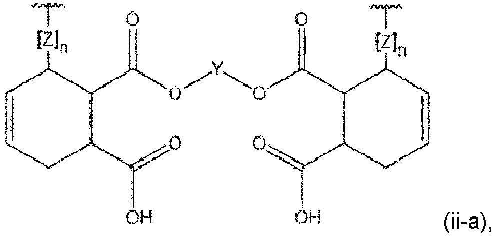 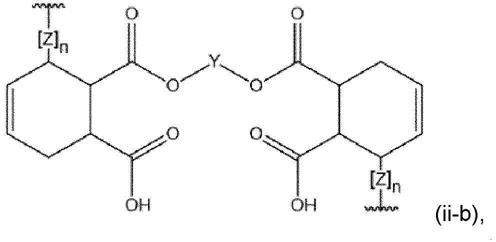 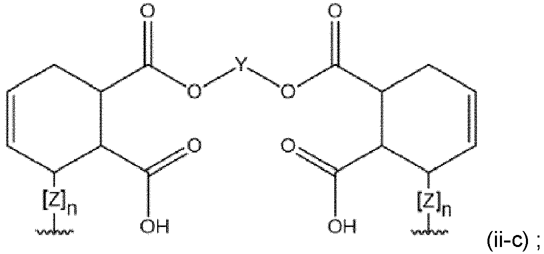 où Z est un groupe de liaison divalent, n vaut 0 ou un nombre entier supérieur à zéro, et Y est un groupe divalent formé à partir d'un polyol ayant au moins deux groupes hydroxyle.

8. La composition réticulée selon la revendication 6 ou la revendication 7, dans laquelle chacun des R⁰, R¹, et R² est un groupe méthyle ; chaque R⁴ est indépendamment R¹ ou un groupe alkyle ayant de 2 à 20 atomes de carbone ; chacun des w et ww est un nombre entier sélectionné indépendamment allant de 50 à 200 ; la somme de x' + x" est un nombre entier allant de 2 à 50 ; la somme de xx' + xx" est un nombre entier allant de 1 à 50 ; et chacun des y et yy est un nombre entier sélectionné indépendamment allant de 1 à 200.

9. La composition réticulée selon l'une des revendications 6 à 8, dans laquelle :
i) chaque R⁴ est indépendamment un groupe alkyle ayant de 4 à 10 atomes de carbone, facultativement de 6 à 8 atomes de carbone ; et/ou
ii) chacun des w et ww est un nombre entier sélectionné indépendamment allant de 75 à 125 ; chacun des x' et xx' est un nombre entier sélectionné indépendamment allant de 2 à 20 ; chacun des x" et xx" est un nombre entier sélectionné indépendamment allant de 1 à 20 ; et chacun des y et yy est un nombre entier sélectionné indépendamment allant de 1 à 20.

10. La composition réticulée selon n'importe laquelle des revendications précédentes, dans laquelle ledit polyol a la formule générale (II) suivante :
HO-R⁵-OH (II)
; dans laquelle R⁵ comprend au moins un élément parmi un groupe hydrocarbylène, hétérohydrocarbylène, ou organohétérylène, facultativement est un groupe hydrocarbylène ayant de 1 à 20 atomes de carbone.

11. La composition réticulée selon n'importe laquelle des revendications précédentes, dans laquelle ledit polyol comprend un polysiloxane ayant au moins deux groupes hydroxyle, facultativement ledit polysiloxane est de la formule générale (III) suivante :
HO-[Z]_{d}-[SiR⁶R⁷-O-]ₐ[SiR⁶R⁸-O-]_{b}[SiR⁶R⁷-O-]_{c}Si-[Z]_{d}-OH (III)
; dans laquelle chacun des R⁶ et R⁷ est un groupe hydrocarbyle substitué ou non substitué sélectionné indépendamment ; R⁸ comprend un groupe hydrocarbyle substitué ou non substitué sélectionné indépendamment ou -[Z]_{d}-OH ; chaque Z comprend indépendamment au moins un élément parmi un groupe hydrocarbylène, hétérohydrocarbylène, ou organohétérylène, facultativement chaque Z est un groupe hydrocarbylène ayant de 1 à 20 atomes de carbone ; a est un nombre entier sélectionné entre 0 et 1 000 ; b est un nombre entier sélectionné entre 1 et 200 ; c est un nombre entier sélectionné entre 0 et 1 000 ; et chaque d vaut indépendamment 0 ou 1, facultativement vaut 1.

12. Une composition réticulée comprenant le produit de réaction :
d'un premier siloxane ayant au moins un groupe anhydride pendant ;
d'un deuxième siloxane ayant au moins un groupe anhydride pendant ; et
d'un polyol ayant au moins deux groupes hydroxyle qui réagissent avec les groupes anhydride pendants desdits premier et deuxième siloxanes ;
dans laquelle au moins l'un des groupes anhydride pendants comprend une liaison insaturée.

13. Une composition pour soins personnels comprenant :
(a) une composition réticulée ayant au moins deux groupes carboxyle ; et
(b) au moins un constituant cosmétique ;
(c) facultativement dans un milieu acceptable d'un point de vue cosmétique ; dans laquelle ladite composition réticulée est telle qu'énoncée dans n'importe laquelle des revendications précédentes.

14. Un procédé de formation d'une composition réticulée, ledit procédé comprenant :
la fourniture d'un organohydrogénopolysiloxane de la formule générale (B) suivante :
R⁰R¹₂Si-O-[SiR¹R²-O-]_{w}[SiHR¹-O-]ₓ[SiR¹R⁹-O-]_{y}SiR¹₂R⁰ (B)
;
dans laquelle chaque R⁰ est un groupe hydrocarbyle substitué ou non substitué sélectionné indépendamment ou un hydrogène, facultativement est un groupe alkyle sélectionné indépendamment ; chacun des R¹ et R² est un groupe hydrocarbyle substitué ou non substitué sélectionné indépendamment, facultativement est un groupe alkyle sélectionné indépendamment ; R⁹ est un groupe hydrocarbyle substitué ou non substitué ou un hydrogène, facultativement est un groupe alkyle, un groupe aryle, ou un groupe polyéther ; w est un nombre entier allant de 0 à 1 000, facultativement de 1 à 300 ; x est un nombre entier allant de 1 à 100, facultativement de 2 à 75 ; et y est un nombre entier allant de 0 à 1 000, facultativement de 1 à 300 ;
la fourniture d'un anhydride polycyclique à fonctionnalité alcényle ;
facultativement, la fourniture d'un alcène linéaire ayant de 2 à 20 atomes de carbone ;
la fourniture d'un polyol ayant au moins deux groupes hydroxyle ;
la combinaison de l'organohydrogénopolysiloxane et de l'anhydride polycyclique à fonctionnalité alcényle, et facultativement de l'alcène linéaire, en présence d'un catalyseur d'hydrosilylation afin de former un intermédiaire de réaction comprenant des siloxanes ayant au moins un groupe anhydride polycyclique pendant ; et
la combinaison des siloxanes et du polyol afin de former la composition réticulée.

15. Une composition de polyorganosiloxane comprenant le produit de réaction :
d'un composé anhydride polycyclique de la formule générale suivante : 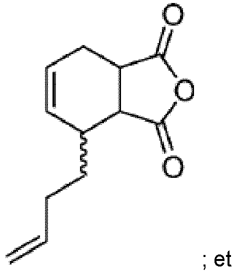 d'un composé d'organosilicium ayant au moins un atome d'hydrogène lié à un silicium.
